(19) Europäisches Patentamt
European Patent Office
Office européen des brevets

(11) **EP 1 152 794 B1**

(12) **EUROPÄISCHE PATENTSCHRIFT**

(45) Veröffentlichungstag und Bekanntmachung des Hinweises auf die Patenterteilung:
**06.10.2004 Patentblatt 2004/41**

(51) Int Cl.[7]: **A61N 5/10**, G01T 1/29

(21) Anmeldenummer: 00905037.8

(22) Anmeldetag: **08.02.2000**

(86) Internationale Anmeldenummer:
**PCT/EP2000/000995**

(87) Internationale Veröffentlichungsnummer:
**WO 2000/048680 (24.08.2000 Gazette 2000/34)**

(54) **VERFAHREN ZUM BETREIBEN EINES IONENSTRAHL-THERAPIESYSTEMS UNTER ÜBERWACHUNG DER BESTRAHLUNGSDOSISVERTEILUNG**

METHOD FOR OPERATING AN ION BEAM THERAPY SYSTEM BY MONITORING THE DISTRIBUTION OF THE RADIATION DOSE

PROCEDE PERMETTANT DE FAIRE FONCTIONNER UN SYSTEME DE THERAPIE A FAISCEAU IONIQUE AVEC SURVEILLANCE DE LA REPARTITION DE LA DOSE DE RAYONNEMENT

(84) Benannte Vertragsstaaten:
**AT BE CH CY DE DK ES FI FR GB GR IE IT LI LU MC NL PT SE**

(30) Priorität: **19.02.1999 DE 19907097**

(43) Veröffentlichungstag der Anmeldung:
**14.11.2001 Patentblatt 2001/46**

(73) Patentinhaber: **Gesellschaft für Schwerionenforschung mbH 64291 Darmstadt (DE)**

(72) Erfinder:
• **HARTMANN, Günther**
  **D-64291 Darmstadt (DE)**
• **HEEG, Peter**
  **D-64291 Darmstadt (DE)**
• **JAEKEL, Oliver**
  **D-64291 Darmstadt (DE)**
• **KARGER, Christian**
  **D-64291 Darmstadt (DE)**

(74) Vertreter: **Boeters, Hans Dietrich, Dr. et al Patentanwälte Boeters & Bauer, Bereiteranger 15 81541 München (DE)**

(56) Entgegenhaltungen:
**WO-A-89/00703**

• **RENNER ET AL: "Preliminary results of a raster scanning beam delivery system" PROCEEDINGS OF THE 1989 IEEE PARTICLE ACCELERATOR CONFERENCE, 20. - 23. März 1989, Seiten 672-674, XP002136858 Chicago**
• **FUKUMURA ET AL: "Carbon beam dosimetry intercomparison at HIMAC" PHYSICS IN MEDICINE AND BIOLOGY, Bd. 43, 1998, Seiten 3459-3463, XP002136859**
• **DAFTARI I K ET AL: "NEW UCSF PROTON OCULAR BEAM FACILITY AT THE CROCKER NUCLEAR LABORATORY CYCLOTRON (UC DAVIS)" NUCLEAR INSTRUMENTS & METHODS IN PHYSICS RESEARCH, SECTION - A: ACCELERATORS, SPECTROMETERS, DETECTORS AND ASSOCIATED EQUIPMENT,NL,NORTH-HOLLAND PUBLISHING COMPANY. AMSTERDAM, Bd. 380, Nr. 3, 11. Oktober 1996 (1996-10-11), Seiten 597-612, XP000683594 ISSN: 0168-9002**
• **CHU W T ET AL: "INSTRUMENTATION FOR TREATMENT OF CANCER USING PROTON AND LIGHT-ION BEAMS" REVIEW OF SCIENTIFIC INSTRUMENTS,US,AMERICAN INSTITUTE OF PHYSICS. NEW YORK, Bd. 64, Nr. 8, 1. August 1993 (1993-08-01), Seiten 2055-2122, XP000393868 ISSN: 0034-6748**

**Beschreibung**

Die vorliegende Erfindung betrifft ein Verfahren zum Betreiben eines Ionenstrahl-Therapiesystems, welches insbesondere mit Schwerionen betrieben wird. WO 89/007 03 offenbart ein Verfahren zum Betreiben eines Strahl-Therapiesystems unter Überwachung der Bestrahlungsdosisverteilung, wobei die Bestrahlungsdosisverteilung an verschiedenen Positionen im Bereich des Isozentrums gemessen und ausgewertet wird. Renner et al., Preliminary results of a raster scanning beam delivery system, Proceedings of the 1989 IEEE Particle accelerator conference, 20-23 März, Seiten 672-674 beschreibt die Überwachung der Bestrahlungsdosisverteilung einer Rasterscannereinrichtung eines Ionenstrahl-Therapiesystems.

Ionenstrahl-Therapiesyteme werden bevorzugt zur Behandlung von Tumoren eingesetzt. Sie besitzen den Vorteil, daß bei Bestrahlung eines Zielobjekts (Targets) der größte Teil der Energie des Ionenstrahls auf das Target übertragen wird, während lediglich eine geringe Energie auf gesundes Gewebe übertragen wird. Demzufolge kann eine relativ hohe Bestrahlungsdosis zur Behandlung eines Patienten eingesetzt werden. Röntgenstrahlen übertragen hingegen ihre Energie gleichermaßen auf das Target sowie gesundes Gewebe, so daß aus gesundheitlichen Gründen zum Schutz des Patienten keine hohe Bestrahlungsdosis verwendet werden kann.

Aus der US-Patentschrift 4,870,287 ist beispielsweise ein Ionenstrahl-Therapiesystem bekannt, bei dem von einer Protonenquelle Protonenstrahlen erzeugt werden, deren Protonen über eine Beschleunigereinrichtung verschiedenen Behandlungs- bzw. Bestrahlungsplätzen zugeführt werden können. An jedem Behandlungsplatz ist ein Drehgerüst mit einer Patientenliege vorgesehen, so daß der Patient mit dem Protonenstrahl unter unterschiedlichen Bestrahlungswinkeln bestrahlt werden kann. Während sich der Patient innerhalb des Drehgerüsts räumlich an einer festen Stelle befindet, rotiert das Drehgerüst um den Körper des Patienten, um die Behandlungsstrahlen unter verschiedenen Bestrahlungswinkeln auf das im Isozentrum des Drehgerüsts befindliche Target zu fokussieren. Die Beschleunigereinrichtung umfaßt die Kombination aus einem linearen Beschleuniger (Linear Accelerator, LINAC) und einem sog. Synchrotronring.

In H.F. Weehuizen et al, CLOSED LOOP CONTROL OF A CYCLOTRON BEAM FOR PROTON THERAPY, KEK Proceedings 97-17, Januar 1998 wird ein Verfahren zur Stabilisierung des Protonenstrahls in Protonenstrahl-Therapiesystemen vorgeschlagen, wobei der Behandlungsstrahl aktiv derart gesteuert wird, daß er an zwei voneinander in Längsrichtung beabstandeten Meßpunkten auf der Mittellinie des entsprechenden Strahlzufuhrsystems liegt. Der erste Meßpunkt liegt zwischen einem Paar von Ablenkmagneten und ist durch eine Vieldraht-Ionisationskammer gebildet. Abhängig von dem von dieser Vieldraht-Ionisationskammer gelieferten Istwert der Strahlposition bezüglich des Mittelpunkts des Strahlpfads wird eine PI-Regelung von weiteren Ablenkmagneten, die vor dem erstgenannten Paar von Ablenkmagneten angeordnet sind, erzeugt. Der zweite Meßpunkt liegt kurz vor dem Isozentrum und ist durch eine in vier Quadranten unterteilte Ionisationskammer gebildet. Abhängig von dem Positionsistwert dieser Ionisationskammer werden wiederum PI-Regelsignale erzeugt, die jedoch für die erstgenannten Ablenkmagnete bestimmt sind. Mit Hilfe dieser Regelung soll sowohl eine Winkelstabilität bezüglich der Mittellinie des Strahlzufuhrsystems als auch eine laterale Positionsstabilität des Protonenstrahls möglich sein.

Bei Durchführung einer Schwerionenbestrahlung, d.h. einer Bestrahlung mit Ionen, die schwerer als Protonen sind, sind jedoch große und schwere Einrichtungen erforderlich, so daß hier die Tendenz besteht, den Einsatz von Drehgerüsten zu vermeiden und statt dessen den Patienten bzw. die Patientenliege zu bewegen. Entsprechende Therapiesysteme sind beispielsweise in E. Pedroni: Beam Delivery, Proc. 1$^{st}$ Int. Symposium on Hadrontherapy, Como, Italy, October 18-21, 1993, Seite 434 beschrieben. Bei diesen Systemen handelt es sich demnach um exzentrische Systeme.

Da jedoch von Onkologen grundsätzlich isozentrische Systeme bevorzugt werden, wurde ein Schwerionenstrahl-Therapiesystem vorgeschlagen, bei dem zwar Drehgerüste an den Behandlungsplätzen eingesetzt werden, jedoch die Radien der Drehgerüste dadurch verringert werden können, daß der jedem Drehgerüst horizontal entlang seiner Drehachse zugeführte Behandlungsstrahl mit Hilfe von geeigneten Magnet- und Optikanordnungen derart geführt wird, daß er zunächst von der Drehachse wegläuft und später wieder die Drehachse im Isozentrum zur Bestrahlung eines Targets kreuzt. Zur Bestrahlung des Targets ist ein Rasterscanner vorgesehen, der vertikale Ablenkmittel sowie horizontale Ablenkmittel umfaßt, die jeweils die Behandlungsstrahlen senkrecht zur Strahlachse ablenken, so daß eine das Target umgebende Fläche mit den Behandlungsstrahlen und einer entsprechenden Bestrahlungsdosisverteilung abgetastet wird. Dieses System sieht somit im wesentlichen eine Strahlführung in lediglich einer Ebene des Drehgerüsts vor.

Da bei Ionenstrahl-Therapiesystemen grundsätzlich eine hohe Betriebssicherheit und Betriebsstabilität hinsichtlich des Behandlungsstrahls erforderlich ist, ist bei dem zuvor beschriebenen Schwerionenstrahl-Therapiesystem eine Überwachungseinrichtung zum Überwachen des von dem Rasterscanner gelieferten Behandlungsstrahls vorgesehen. Diese Überwachungseinrichtung ist zwischen dem letzten Ablenkmagneten der oben genannten Magnetanordnung und dem Isozentrum angeordnet und kann Ionisationskammern zur Überwachung des Teilchenflusses und vieldrahtkammern zur Überwachung der Strahlposition und der Strahlbreite umfassen.

Beim Betrieb von medizinischen Elektronenbeschleunigern sind aus Sicherheitsgründen verschiedene DIN-Normen einzuhalten. Diese betreffen zum einen die Abnahmeprüfung, d.h. die Überprüfung der Betriebsbereitschaft, und zum anderen die Konstanzprüfung, d.h. die Überprüfung der Betriebsstabilität, des Systems. Für Ionenstrahl-Therapiesysteme, insbesondere für Schwerionenstrahl-Therapiesysteme, sind derartige eigens für Ionenstrahl-Therapiesystem entwickelte Sicherheitsnormen noch nicht bekannt. Auch bei Ionenstrahl-Therapiesystemen besteht jedoch das Bedürfnis nach einer größtmöglichen Betriebssicherheit und Betriebsstabilität.

Der vorliegenden Erfindung liegt daher die Aufgabe zugrunde, ein Verfahren zum Betreiben eines Ionenstrahl-Therapiesystems vorzuschlagen, wobei eine ausreichende Betriebssicherheit und Betriebsstabilität bezogen auf die Bestrahlungdurchführung gewährleistet ist. Dabei soll das Verfahren insbesondere zur Verwendung mit Schwerionen geeignet sein.

Diese Aufgabe wird gemäß der vorliegenden Erfindung durch ein Verfahren mit den Merkmalen des Anspruches 1 gelöst. Die abhängigen Ansprüche definieren jeweils bevorzugte und vorteilhafte Ausführungsformen der vorliegenden Erfindung.

Die vorliegende Erfindung betrifft ein Verfahren zum Betreiben eines Ionenstrahl-Therapiesystems, welches eine in einem Strahlführungssystem angeordnete Rasterscannereinrichtung mit vertikalen Ablenkmitteln und horizontalen Ablenkmitteln zur vertikalen bzw. horizontalen Ablenkung eines Behandlungsstrahls senkrecht zu seiner Strahlrichtung umfaßt, so daß der Behandlungsstrahl von der Rasterscannereinrichtung auf ein Isozentrum des Bestrahlungsplatzes abgelenkt wird und eine bestimmte, das Isozentrum umgebende Fläche mit einer bestimmten Bestrahlungsdosis abtastet. Vorzugsweise wird sowohl die Tiefendosisverteilung als auch die Dosisquerverteilung der Rasterscannereinrichtung an verschiedenen Positionen im Bereich des Isozentrums gemessen und ausgewertet, wobei auf eine ausreichend homogene Bestrahlungsdosisverteilung geschlossen wird, falls die Schwankungsbreite der an den einzelnen Positionen gemessenen Bestrahlungsdosiswerte einen bestimmten Toleranzgrenzwert nicht überschreitet.

Ferner wird gemäß der Erfindung die zeitliche Schwankung von Kalibrierfaktoren von Überwachungsmitteln, welche zur Überwachung bestimmter Strahlparameter des Behandlungsstrahls eingesetzt werden, den Einfluß der Teilchenfluenz oder des Teilchenflusses des Behandlungsstrahls auf diese Kalibrierfaktoren, die Abhängigkeit der Kalibrierfaktoren von der Strahllage des Behandlungsstrahls sowie die Konstanz der Bestrahlungsdosis überwacht, wobei jeweils entsprechende Toleranzgrenzen im Sinne von Interventionsschwellen definiert werden.

Die vorliegende Erfindung definiert ein umfassendes Prüfschema für Ionenstrahl-Therapiesysteme.

Die vorliegende Erfindung ermöglicht somit eine deutliche Verbesserung der Betriebsstabilität und Betriebssicherheit des Ionenstrahl-Therapiesystems, wobei die einzelnen Prüfaspekte des Prüfschemas im Sinne einer Abnahmeprüfung und/oder einer Konstanzprüfung des Ionenstrahl-Therapiesystems durchgeführt werden können. Dies betrifft insbesondere die Überprüfung von Merkmalen des Ionenstrahl-Therapiesystems, welche den Rasterscanvorgang und den Bereich der Dosimetrie betreffen.

Die Erfindung wird nachfolgend anhand eines bevorzugten Ausführungsbeispiels unter Bezugnahme auf die beigefügte Zeichnung beschrieben.

Fig. 1 zeigt eine vereinfachte schematische Darstellung einer bei dem vorliegenden Ionenstrahl-Therapiesystem eingesetzten Beschleunigungseinrichtung,

Fig. 2 zeigt eine Ansicht eines bei dem vorliegenden Ionenstrahl-Therapiesystem eingesetzten Drehgerüsts, und

Fig. 3A und 3B sowie Fig. 4A und 4B zeigen jeweils Darstellungen, welche die vorteilhaften Auswirkungen von Strahlpositions-Regelungsmaßnahmen bei den in Fig. 1 und 2 gezeigten Ionenstrahl-Therapiesystem verdeutlichen.

Ein der vorliegenden Erfindung zugrundeliegendes Ionenstrahl-Therapiesystem wird in der Regel in Krankenhausgebäuden eingesetzt, welche in einen medizinischen Bereich und einen Beschleunigerbereich unterteilt sind. zur Behandlung von Patienten sind mehrere Behandlungs- oder Bestrahlungsplätze vorgesehen. Das Kontrollsystem des Ionenstrahl-Therapiesystems umfaßt mehrere Kontrollräume, wobei für die einzelnen Behandlungsplätze technische Kontrollräume und ein Hauptkontrollraum für die Beschleunigereinrichtung vorgesehen sein können. Darüber hinaus können in dem Gebäude Laboratorien für die Dosimetrie oder zur Beschleunigerwartung oder eine PET-Einrichtung (Positron-Emitter-Tomograph) untergebracht sein. Zudem sind Energieversorgungseinrichtungen (insbesondere für die Beschleunigereinrichtung und das Bestrahlungssystem) und Kühleinrichtungen vorgesehen. Die einzelnen Behandlungsräume sind durch dicke Wände und Decken begrenzt, die beispielsweise aus Beton mit einer Dicke von 2 m bestehen, um eine ausreichende Abschirmwirkung sicherzustellen.

Da der Grundaufbau des Ionenstrahl-Therapiesystems im wesentlichen nicht Thema der vorliegenden Erfindung ist, wird an dieser Stelle lediglich kurz darauf eingegangen.

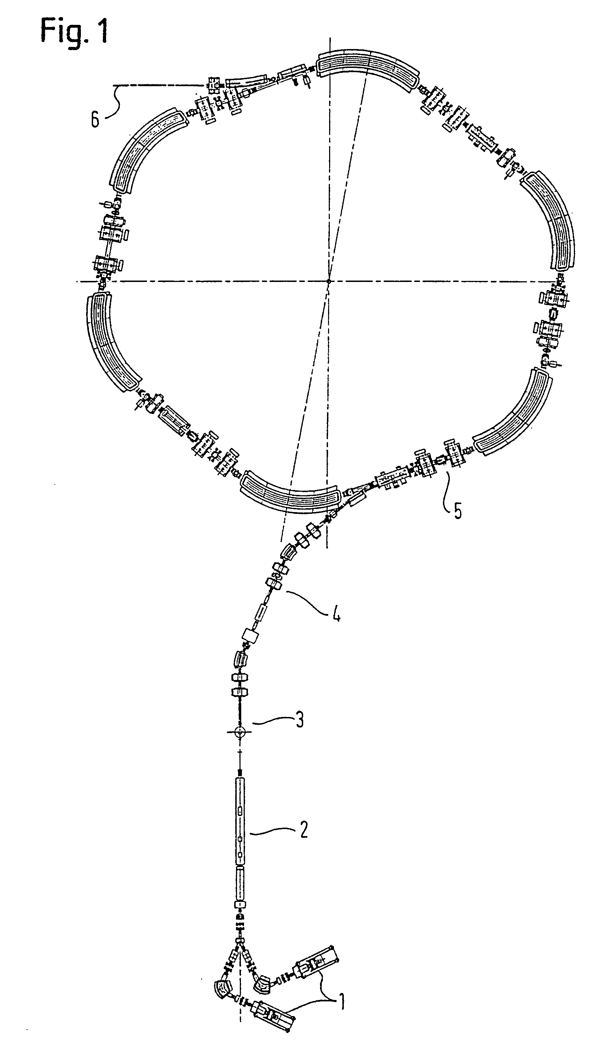

Das Ionenstrahl-Therapiesystem umfaßt ein Injektionssystem, welches zusammen mit der bereits zuvor erwähnten Beschleunigereinrichtung in Fig. 1 vereinfacht dargestellt ist.

Das Injektionssystem umfaßt Ionenquellen 1, deren Strahlung jeweils über Niederenergie-Strahlführungsleitungen mit einer Anordnung von Spektrometermagneten und Quadrupolen einem Schaltmagneten zugeführt werden, welcher die Strahlung u.a. über eine weitere Quadrupolanordnung und eine zur Pulsformung vorgesehenen Chopperanordnung einem linearen Beschleuniger 2 (Linear Accelerator, LINAC) zuführt.

Bei dem vorliegenden Ausführungsbeispiel sollen ausschließlich $^{12}C^{2+}$-Ionen verwendet werden, die in der Strahlführung zwischen dem linearen Beschleuniger 3 und dem Synchrotronring 5 auf $^{12}C^{6+}$ gestrippt werden. Zu diesem Zweck ist nach dem linearen Beschleuniger 2 ein Stripper 3 vorgesehen. Diese Kohlenstoffionen haben sich aufgrund ihrer physikalischen und biologischen Eigenschaften als sehr effektiv bei der Behandlung von Tumoren herausgestellt und besitzen die Vorteile einer hohen physikalischen Selektivität sowie einer hohen biologischen Effektivität und bieten darüber hinaus die Möglichkeit, die Bestrahlung mit Hilfe eines Positron-Emitter-Tomographen (PET) zu verifizieren. Durch die geeignete Auswahl der Kohlenstoffionen kann die biologische Effektivität derart gesteuert werden, daß sie im Plateaubereich der Bragg'schen Kurve gering und im Bereich des Bragg-Peaks hoch ist. Dadurch kann das Target bzw. der Tumor mit einer höheren Dosis behandelt werden, während die Dosis für das umgebende gesunde Gewebe minimiert wird.

Um die Verwendung bzw. Beschleunigung ausschließlich der beabsichtigten Ionensorte sicherzustellen, wird in dem Hochladungs-Injektionssystem ein Ladungsspektrum des vorliegenden Strahls aufgenommen und ausgewertet. Durch Vergleich des aufgenommenen Ladungsspektrums mit einem Referenzspektrum können ungewünschte Ionen oder Störstellen erkannt und entsprechende Maßnahmen ergriffen werden. Diese Überprüfung kann beispielsweise mit jedem Hochfahren einer Ionenquelle 1 durchgeführt werden.

Der lineare Beschleuniger 2 dient zur ersten Beschleunigung der ihm zugeführten Ionen, welche anschließend über eine Injektionsleitung 4 dem Synchrotron 5 zugeführt werden. Die Injektionsleitung 4 umfaßt neben dem bereits erwähnten Stripper 3 eine weitere Chopperanordnung zur Feinformung der Injektionspulse, Dipolmagnete zur Ladungsanalyse, Quadrupole zur Anpassung der Strahlung an das Aufnahmevermögen des Synchrotrons 5 etc..

Das Injektionssystem, welches u.a. die Ionenquellen 1, die Niederenergie-Strahlführungsleitungen, den linearen Beschleuniger 2 (LINAC), den Stripper 3 und die Injektionsleitung 4 umfaßt, besitzt somit insgesamt die Aufgabe, Ionenstrahlen mit gewünschten Teilchen zu erzeugen und zu analysieren, die Verunreinigung der Ionenstrahlen zu überwachen und die Ionenstrahlintensität zu steuern, die Ionen auf eine bestimmte Injektionsenergie zu beschleunigen sowie die Pulslänge der in den Synchrotronring 5 injizierten Pulse zu bestimmen.

Der Synchrotronring 5 dient zu abschließenden Beschleunigung der ihm zugeführten Ionen auf eine bestimmte Energie und umfaßt beispielsweise mehrere Ablenkmagnete, Quadrupole und Sextupole. Bei dem in Fig. 1 gezeigten Ausführungsbeispiel sind beispielsweise sechs Ablenkmagnete mit einem Ablenkwinkel von jeweils 60° vorgesehen. Innerhalb des Synchrotrons 5 ist eine (nicht gezeigte) Kühleinrichtung angeordnet. Durch mehrfache Injektionsumläufe werden die injizierten Ionen von einer Energie im Bereich von einigen MeV/u auf eine Energie von beispielsweise über 400 MeV/u beschleunigt. Der auf diese Weise beschleunigte Behandlungsstrahl wird an einer bestimmten Stelle des Synchrotrons über eine Hochenergie-Strahlführungsleitung 6 extrahiert und den einzelnen Behandlungsplätzen zugeführt.

Obwohl im allgemeinen die horizontale und vertikale Strahlausbreitung am Behandlungsplatz unterschiedlich ist, kann den Forderungen nach einer "idealen" symmetrischen und stabilen Strahlform am Behandlungsplatz im wesentlichen durch eine geeignete Einstellung der Strahloptik in den Strahlführungsleitungen Rechnung getragen werden.

Die Hochenergie-Strahlführungsleitung 6 umfaßt Quadrupollinsen, Ablenkmagnete, Strahlanalyseeinrichtungen usw.. Darüber hinaus kann ein weiterer Chopper hinter der Extraktionsstelle des Synchrotrons 5 angeordnet sein, der in Notfällen zum Einsatz kommt, um die Strahlzufuhr zu unterbrechen. Daneben kann eine routinemäßige Unterbrechung des Extraktionsvorgangs, der zum Auskoppeln des Behandlungsstrahls aus dem Synchrotron 5 dient, nach jedem Rasterscanabschnitt vorgesehen sein.

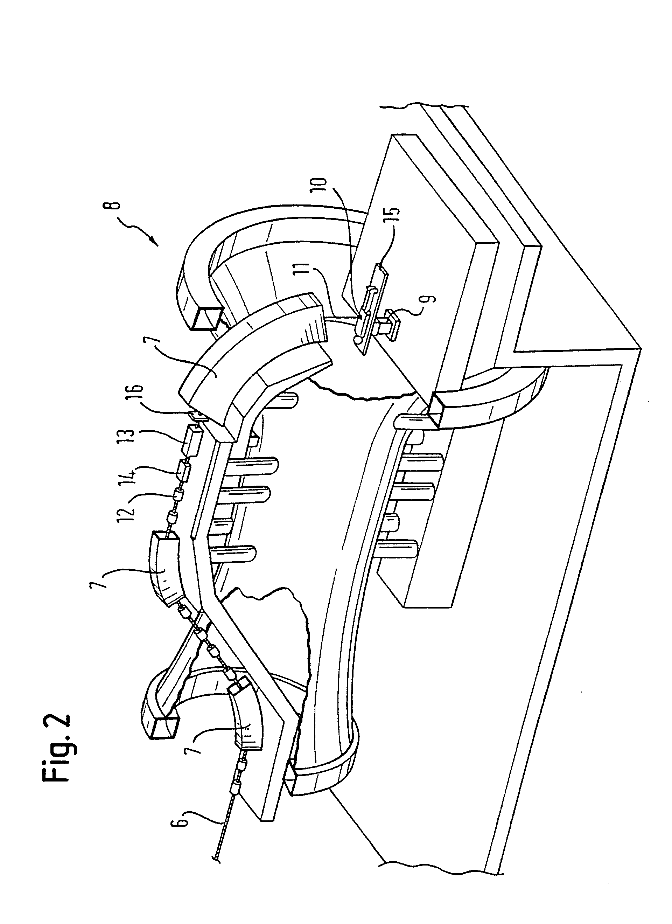

Fig. 2 zeigt eine perspektivische Ansicht eines der Drehgerüste 8, die jeweils an einem der Behandlungsplätze vorgesehen sind, denen der Behandlungsstrahl über die zuvor beschriebene Hochenergie-Strahlführungsleitung 6 zugeführt wird. Das Drehgerüst 8 rotiert um eine bestimmte Drehachse, während ein zu behandelnder Patient auf einer Patientenliege 9 mit örtlich fester Orientierung bzw. Ausrichtung liegt. Die zu behandelnde Körperstelle des Patienten befindet sich dabei im Isozentrum 10 des Behandlungsstrahls, wobei das Isozentrum als Schnittpunkt zwischen dem Zentralstrahl 11 des nachfolgend noch näher beschriebenen Rasterscanners und einer Drehachse der Patientenliege 9 definiert ist.

Wie aus Fig. 2 ersichtlich ist, ist die Hochenergie-Strahlführungsleitung 6 derart ausgestaltet, daß der Behandlungsstrahl nach Eintritt in das Drehgerüst 8 in einer Ebene mehrmals abgelenkt wird. Zu diesem Zweck sind mehrere Quadrupollinsen 12 und Dipolmagnete 7 vorgesehen, wobei die ersten beiden Dipolmagnete 7 identische Ablenkwinkel, beispielsweise 42°, aufweisen und zueinander entgegengesetzt angeordnet sind, während es sich bei dem letzten Dipolmagnet 7 um einen Ablenkmagnet mit einem Ablenkwinkel von 90° handelt, so daß der Behandlungsstrahl 11 nach Eintritt in das Drehgerüst 8 zunächst aus der Drehachse des Drehgerüsts 8 seitlich abgelenkt und anschließend parallel zur Drehachse des Drehgerüsts 8 geführt wird, um anschließend aus dem letzten Ablenkmagneten 7 über eine Strahlaustrittsöffnung unter einem Winkel von 90° bezüglich der Patientenliege 9 auszutreten.

Die bei dem vorliegenden Ionenstrahl-Therapiesystem vorgesehene Rasterscanneranordnung ist bei dem in Fig. 2 gezeigten Ausführungsbeispiel zwischen der letzten Quadrupollinse 12 und dem letzten Ablenkmagneten 7 des Drehgerüsts 8 angeordnet und umfaßt mindestens einen horizontalen Rasterscannermagneten 13 sowie mindestens einen vertikalen Rasterscannermagneten 14. Die Rasterscannermagnete 13 und 14 lenken den Ionenstrahl 11 jeweils senkrecht zur Strahlachse 11 entweder horizontal oder vertikal ab, so daß der auf diese Weise gerasterte Ionenstrahl 11 nach Austritt aus dem letzten Ablenkmagneten 7 in Übereinstimmung mit einem vorgegebenen Behandlungsplan eine bestimmte, das Isozentrum 10 umgebende Fläche abtastet. Aufgrund der Anordnung des Rasterscanners 13, 14 zwischen dem letzten Quadrupolmagneten 12 und dem letzten Ablenkmagneten 7 kann eine hohe Flexibilität bei der nachfolgend noch näher beschriebenen Regelung der Strahlgröße und Strahldispersion am Isozentrum 10 erzielt werden.

Die Rasterscannermagnete 13, 14 werden von einer (nicht gezeigten) Steuereinrichtung angesteuert, welche Bestandteil des Gesamtkontrollsystems des Ionenstrahl-Therapiesystems ist.

Im Bereich zwischen der Strahlaustrittsöffnung des letzten Ablenkmagneten 7 und dem Isozentrum 10 sind Überwachungsmittel zur Überwachung des Behandlungsstrahls 11 vorgesehen. Diese Überwachungsmittel, welche beispielsweise zur Erfassung und Regelung der Strahlposition, Strahlform und des Teilchenflusses vorgesehen sind, werden nachfolgend noch ausführlich erläutert.

Wie bereits zuvor erwähnt worden ist, kann zusätzlich ein Positron-Emitter-Tomograph (PET) zur Überwachung des Bestrahlungsvorgangs vorgesehen sein, dessen Bildaufnehmer (Kamera) in einer In-Strahl-Position ausgerichtet ist. Die Positron-Emitter-Tomographie wird vorzugsweise während der Behandlung bzw. Bestrahlung durchgeführt. Trifft ein Behandlungsstrahl auf Gewebe, werden ausgehend von den Primärionen Positronen emittierende Isotope erzeugt. Einige dieser Isotope, welche sich von den Primärionen lediglich durch den Verlust von ein oder zwei Neutronen unterscheiden, stoppen nahezu in demselben Bereich wie die entsprechenden Primärionen. Dieser Stoppunkt der sogenannten Positronenemitter kann zur Überwachung des Bestrahlungsvorgangs mit Hilfe der Positron-Emitter-Tomographie ermittelt werden.

Für das zuvor beschriebene Ionenstrahl-Therapiesystem wurde ein umfangreiches Prüfsystem zur Überprüfung und Regelung der wesentlichen Leistungsmerkmale des Therapiesystems entwickelt, auf welches nachfolgend näher eingegangen werden soll.

Ein erster Abschnitt des Prüfsystems betrifft die Erzeugung des Behandlungsstrahls 11.

Neben der bereits zuvor beschriebenen Überprüfung der Ionensorte wird dabei auch die Strahlenergie des Behandlungsstrahls überwacht. Dies ist erforderlich, da die von der jeweiligen Therapie geforderten Strahlenergien eingehalten werden müssen. Zu diesem Zweck umfassen die in Fig. 2 angedeuteten Überwachungsmittel ein Absorber-Ionisationskammersystem, welches am Isozentrum 10 des jeweiligen Behandlungsplatzes anzuordnen ist. Das Absorber-Ionisationskammersystem mißt für einige ausgewählte Energiestufen, welche während eines Therapie-Testzyklus aktiviert werden, die Lage des Bragg-Peaks am Behandlungsplatz, wobei sich die augenblickliche Strahlenergie aus der gemessenen Position des Bragg-Peaks ergibt. Zur Bestimmung der Position des Bragg-Peaks werden die Bragg'schen-Kurven in feinen Schritten ausgemessen. Sollte sich bei der Überprüfung eine Abweichung des Bragg-Peaks von der Sollposition um mehr als 0,5 mm ergeben, ist eine Intervention erforderlich. Zu Konstanzprüfung kann der zuvor beschriebene Prüfvorgang vor jedem Bestrahlungsblock durchgeführt werden.

Ein weiterer Unterpunkt hinsichtlich der Überprüfung des Behandlungsstrahls betrifft die Überwachung des Intensitätsniveaus des langsam extrahierten Behandlungsstrahls am Bestrahlungs- bzw. Behandlungsplatz. Die limitierte Dynamik des Rasterscanners begrenzt die Abtast- oder Scangeschwindigkeit des gerasterten Behandlungsstrahls nach oben, wobei die für diese Limitierung maßgebliche Komponente die maximale Stromanstiegsgeschwindigkeit der Magnetstromversorgungseinrichtungen ist. Die Scangeschwindigkeit des Behandlungsstrahls hängt von der jeweiligen Intensität des Strahls und der geplanten Teilchenbelegung ab. Um sicherzustellen, daß die maximale Scangeschwindigkeit während der Bestrahlung nicht erreicht wird, darf die aus dem Synchrotron 5 extrahierte Teilchenrate die Sollvorgabe nicht wesentlich überschreiten. Bei einer deutlichen Unterschreitung hingegen verlängert sich die Gesamtbestrahlungszeit, wobei gegebenenfalls dann das Kontroll- bzw. Überwachungs- oder Monitorsystem im Bereich sehr kleiner Eingangsströme betrieben wird, was eine Beeinträchtigung der Präzision des Strahlnachweises zur Folge haben kann. Daher ist beim vorliegenden Therapiesystem eine Messung und Protokollierung der Teilchenintensitäten im Synchrotron im oberen Intensitätsbereich sowie der dem Bestrahlungsplatz zugeführten Teilchenrate für alle Intensitätsschritte für mehrere Energie über einige Minuten vorgesehen. Die von dem Beschleuniger dem Bestrahlungsplatz zugeführte Teilchenrate liegt zwischen $2 \times 10^6$ und $2 \times 10^8$ Ionen pro Extraktion aus dem Synchrotron 5. Die Abweichung der Teilchenrate von der Sollvorgabe darf nach oben maximal 30 % und nach unten maximal 50 % betragen. Werden diese Grenzwerte überschritten, ist eine entsprechende Intervention erforderlich.

Zur Konstanzprüfung des Therapiesystems kann diese Überprüfung beispielsweise täglich durchgeführt werden.

Bei der Datenversorgung der Beschleuniger, der Bestrahlungsplanung und der Rasterscan-Programmierung müssen die gleichen Abhängigkeiten der Energie-, Intensitäts- und Fokussierungsvariation zugrunde gelegt werden. Um dies sicherzustellen, sollten die nach der letzten Therapie-Programmierung beschleunigerseitig erstellten Dateieinträge mit denjenigen verglichen werden, die für die Rasterscan-Programmierung und Bestrahlungsplanung verwendet werden. Eine Abweichung dieser Dateieinträge ist nicht zulässig. Zur Konstanzprüfung sollte diese Überprüfung vor jedem Bestrahlungsblock durchgeführt werden.

Während einer Bestrahlung werden die für die Therapie erforderlichen Beschleunigerabschnitte gegen (externe) Eingriffe verriegelt, um bewußte und unbewußte Fehleinstellungen zu vermeiden. Gleichzeitig werden Betriebszustände aller Komponenten aktiviert und ausschließlich auf die in Speichern, beispielsweise EPROMS, abgelegten Gerätesolldaten zugegriffen. Die Funktion dieser Beschleuniger-Verriegelung gegen Eingriffe kann dadurch überprüft werden, daß ein "Superzyklus" erstellt wird, der sowohl Experiment- als auch Therapiebeschleuniger enthält. In die Hochenergie-Strahlführung 6 zum Drehgestell 8 werden Überwachungsmittel oder Detektoren, wie z.B. (nachfolgend noch näher beschriebene) Profilgitter, Leuchttargets und Ionisationskammern, eingefahren und strahlbeeinflussende Elemente der Hochenergie-Strahlführungsleitung 6 und des Synchrotrons 5 für den Therapiebeschleuniger deaktiviert. Anschließend wird die Beschleunigerverriegelung aktiviert und alle Experimentbeschleuniger deaktiviert, während der Therapiebeschleuniger aktiviert wird. Zudem werden für den Therapiebeschleuniger alle zuvor deaktivierten Komponenten aktiviert und die eingefahrenen Profilgitter, Leuchttargets und Ionisationskammern wieder hinausgefahren. Nunmehr werden an einzelne Magnete Ausschaltbefehle und an Strahlführungs-Diagnosekomponenten Stellbefehle abgesendet, die im Normalfall aufgrund der Verriegelung des Beschleunigers keine Auswirkungen haben dürfen. Ansonsten liegt ein Fehler vor, der entsprechend korrigiert werden muß. Diese Überprüfung kann zur Konstanzprüfung vor jedem Bestrahlungsblock durchgeführt werden.

Die Extraktion des Behandlungsstrahls aus dem Synchrotron 5 muß aus Sicherheitsgründen innerhalb weniger als 1ms nach einem entsprechenden Signal einer Interlockeinheit des Therapiesystems abgebrochen werden können. Dies geschieht durch ein schnelles Abschalten eines speziellen Quadrupols im Synchrotron 5. Die Zeit zwischen einer Anforderung zum Strahlabbruch durch das Kontroll- und Sicherheitssystem und dem Ausbleiben des Strahls am Bestrahlungsort ist von entscheidender Bedeutung sowohl für den Rasterscanvorgang beim Wechsel aufeinanderfolgender Isoenergieabschnitte, welche mit konstanter Energie zu bestrahlenden Flächen entsprechen, als auch für eine eventuelle Notabschaltung des Systems im Fehlerfall. Es ist daher ein Test vorgesehen, der die Gesamtzeit, d.h. sowohl die Reaktionszeit der Anforderung als auch die des Strahlabbruchs, mißt. zu diesem Zweck erzeugt das Kontrollsystem ein entsprechendes Signal, welches die Beendigung eines Isoenergieabschnitts simuliert, oder es wird eine Interlockbedingung, d.h. eine Bedingung für eine Notabschaltung, erzeugt. Anschließend wird von dem Kontrollsystem die Teilchenzahl nach einem Abbruch gemessen, wobei diese 1 ms nach dem Abbruch nicht größer als $10^4$ Teilchen/s sein darf. Zusätzlich wird mit einem Speicheroszillographen und einem Pulser, welche in dem technischen Kontrollraum des Therapiesystems fest installiert sind, eine Messung durchgeführt, die das Ausgangssignal des Strom-Spannungs-Wandlers einer der Ionisationskammern auswertet, um somit die zuvor beschriebene Messung des Kontrollsystems zu überprüfen. Auch bei dieser zweiten Messung darf somit 1 ms nach Abbruch kein Strahl nachgewiesen werden können. Folgende Zeitpunkte eines Abbruchs sollten nacheinander überprüft werden: zu Beginn der Extraktionszeit, in der Mitte der Extraktionszeit, am Ende der Extraktionszeit und außerhalb der Extraktionszeit. Die Überprüfung sollte als Konstanzprüfung täglich durchgeführt werden.

Nach Beendigung jeder Bestrahlung muß von der Beschleunigerseite ein Protokoll erstellt werden, welches die Einstellungen wesentlicher Beschleunigerkomponenten während der Bestrahlung sowie ausgewählte Strahldiagnose-Meßergebnisse dokumentiert. Um die Funktionsfähigkeit der Protokollierung sowie den Protokollinhalt zu testen, wird vorgeschlagen, einen Referenz-Therapiezyklus zu aktivieren und das Protokollprogramm aufzurufen. Anschließend können die durch das Protokollprogramm erstellten Protokolldaten mit den erwarteten Daten verglichen werden, wobei bei Unvollständigkeit des Protokolls oder bei Vorliegen eines protokollierten Gerätefehlers eine Intervention erfolgen muß. Zur Konstanzprüfung kann dieser Prüfvorgang vor jedem Bestrahlungsblock durchgeführt werden.

Ein zweiter Abschnitt des Prüfsystems betrifft die Überprüfung der Führung des Behandlungsstrahls (vor dem Bestrahlungsplatz).

Vom Beschleuniger aus muß sichergestellt werden, daß bei einer Abbruchanforderung ein Extraktionsabbruch erfolgt ist. Sollte der Behandlungsstrahl nicht durch die Abbruchanforderung abgebrochen werden, wird dies von dem Kontroll- und Sicherheitssystem durch eine Intensitätsmessung festgestellt und über einen redundanten, getrennt vorgesehenen Kanal erneut ein Abbruch des Strahls angefordert. Diese zweite Anforderung wirkt auf einen entsprechenden Ablenkdipol der Hochenergie-Strahlführungsleitung 6. Um auch die Funktionsfähigkeit dieses redundanten Extraktionsabbruchs zu überprüfen, wird die für den ersten Extraktionsabbruch vorgesehene Alarmleitung künstlich unterbrochen. In diesem Fall müßte der zuvor beschriebene zweite Extraktionsabbruch automatisch ausgelöst werden, was analog zu der oben beschriebenen Überprüfung des eigentlichen Extraktionsabbruchs getestet werden kann. Kommt es innerhalb von 10 ms nicht zu einem Extraktionsabbruch, ist ein entsprechender Eingriff erforderlich. Zur Konstanzprüfung kann dieser Test vor jedem Bestrahlungsblock durchgeführt werden.

Mit einem weiteren Test kann die Funktion der Zu- und Ausschaltung der in der Hochenergie-Strahlführungsleitung 6 angeordneten Dipole getestet werden. Aus Gründen der Patientensicherheit ist die Ausschaltung der letzten beiden Ablenkmagnete in der Hochenergie-Strahlführungsleitung 6 vor der Bestrahlung (nach der Beschleunigerverriegelung) nur von dem technischen Kontrollraum aus über spezielle Kabelverbindungen zum Netzgerät dieser Magnete aktivierbar. Durch diese Ausschaltung wird die Strahlzufuhr an den Bestrahlungsplatz unterbunden. Die Zuschaltung dieser Magnete kann nur von dem technischen Kontrollraum aus über ein spezielles Signal und nicht (wie üblich) vom Hauptkontrollraum des Beschleunigers aus erfolgen. Die Funktion dieser zu- und Abschaltung wird getestet, wobei dabei auch die entsprechenden Verbindungen/Anschlüsse überprüft werden. Zur Konstanzprüfung wird dieser Test vor jedem Bestrahlungsblock durchgeführt.

Ein dritter Abschnitt des Prüfsystems betrifft die Überprüfung der Strahlführung am Bestrahlungsplatz.

Gemäß einem ersten Aspekt dieses Prüfabschnitts wird die Nullpunktslage des Behandlungsstrahls überwacht. Um eine genaue Strahlpositionierung am Isozentrum 10 nach Ablenkung des Strahls 11 durch die Rasterscannermagnete 13, 14 zu gewährleisten, muß die Achslage des Behandlungsstrahls 11 im letzten Teil der Strahlführung zum Bestrahlungsplatz für den gesamten Energie- und Fokussierungsbereich überprüft werden. Zu diesem Zweck werden Profilgitter 16 hinter den Rasterscannermagneten 13 und 14 sowie am Strahlaustrittsfenster in den Strahlpfad eingefahren und Testzyklen über den gesamten Energie- und Fokussierungsbereich erzeugt. Dabei werden die Profilgitter einzeln ausgewertet und die dabei erfaßten Strahlparameter protokolliert. Bei der Messung des am Strahlaustrittsfenster angeordneten Profilgitters muß das davor angeordnete Profilgitter 16 herausgefahren werden. Durch Auswertung der von den Profilgittern gelieferten Strahlparameter kann sowohl in horizontaler als auch in vertikaler Richtung die Strahlposition und der Strahlwinkel bestimmt werden. Aus den Strahlpositionen der Profilgitter wird die zu erwartende Position des Behandlungsstrahls am Isozentrum 10 ermittelt und anschließend das Protokoll überprüft. Wird dabei für das Isozentrum 10 ein Positionsfehler von $\pm 25$ % bezüglich der geforderten Strahl-Halbwertsbreiten festgestellt, muß ein entsprechender Eingriff erfolgen. Zu Konstanzprüfung kann dieser Test täglich durchgeführt werden.

Gemäß einem weiteren Aspekt dieses Prüfabschnitts wird der absolute Strahlort sowie die Ortsstabilität des Behandlungsstrahls am Bestrahlungsplatz überprüft. Die Einhaltung der absoluten Strahlposition ist die Voraussetzung für die Umsetzung der Behandlungs- bzw. Bestrahlungspläne. Daher muß der absolute Ort mit ortsempfindlichen Detektoren des Kontrollsystems gemessen werden. Die relative Ortsstabilität des Behandlungsstrahls im Isozentrum des Bestrahlungsplatzes bestimmt die Genauigkeit, mit der ein Bestrahlungsplan durchgeführt werden kann. Der Ort des Behandlungsstrahls wird online, d.h. kontinuierlich, während einer Bestrahlung gemessen und überprüft. Bei Abweichungen vom Sollort innerhalb einer vom Bestrahlungsplan vorgegebenen Toleranzgrenze wird die Bestrahlung abgebrochen bzw. eine entsprechende Intervention getätigt. Jeder ortsempfindliche Detektor wird getrennt überprüft.

Die Prüfung wird mit einem Profilgitter und ortsempfindlichen Detektoren, wie z.B. Vieldrahtkammern, durchgeführt.

Bei Verwendung von Profilgittern wird die absolute Strahllage im Isozentrum 10 mit Hilfe eines Leuchttargets oder Films am Ort des Isozentrums überprüft. Dabei wird die Lage des Profilgitters mit dem durch ein Laserkreuz auf dem Leuchttarget oder Film sichtbar gemachten Isozentrum justiert. Mit den Rasterscannermagneten 13, 14 wird der Behandlungsstrahl 11 statisch in das Isozentrum 10 abgelenkt und die durch die Profilgittermessung erhaltenen Ortskoordinaten mit den vorgegebenen Sollwerten verglichen. Dies kann beispielsweise in regelmäßigen Abständen, z.B. bei etwa jedem zehnten Energieschritt, durchgeführt werden.

Bei Verwendung von Vieldrahtkammern zur online-Kontrolle und Regelung der Strahllage werden zwei Vieldrahtkammern in einem Abstand von etwa 970 mm und 790 mm vor dem Isozentrum 10 positioniert und mit Hilfe eines Laserstrahls derart ausgerichtet, daß der durch das Isozentrum 10 verlaufende Zentralstrahl senkrecht durch das Zentrum der Vieldrahtkammern verläuft. Mit den Rasterscannermagneten 13, 14 wird der Strahl beispielsweise bei fünf verschiedenen Energien auf jeweils fünf unterschiedliche Positionen innerhalb des Bestrahlungsbereichs (nämlich oben und unten jeweils links und rechts sowie in der Mitte) statisch abgelenkt. Der Ort der Einstellung wird von dem Kontrollsystem gemessen und mit den Sollwerten verglichen.

Da sich die Vieldrahtkammern in unterschiedlicher Entfernung vor dem Isozentrum befinden, wird das Bestrahlungsfeld in den beiden Vieldrahtkammern mit unterschiedlichen Faktoren verkleinert abgebildet. Durch Anwendung der Regeln der Strahlgeometrie und des Strahlensatzes ergeben sich folgende Verkleinerungsfaktoren:

Vieldrahtkammer 970 mm vor dem Isozentrum:

    x-Koordinate: Verkleinerungsfaktor 0,890
    y-Koordinate: Verkleinerungsfaktor 0,876

Vieldrahtkammer 790 mm vor dem Isozentrum:

    x-Koordinate: Verkleinerungsfaktor 0,910
    y-Koordinate: Verkleinerungsfaktor 0,899

Vor der Überprüfung der absoluten Strahlposition mit den vieldrahtkammern sollte eine Kalibrierung ihrer absoluten Positionen durchgeführt werden. Zu diesem Zweck wird nach Ausrichtung und Fixierung der Vieldrahtkammern ein mittels des zuvor erwähnten Laserkreuzes absolut positionierter Film an fünf Positionen bestrahlt. Der anhand des Films ermittelte Strahl-Nullpunkt wird mit dem aus den Vieldrahtkammern berechneten verglichen. Die Differenz oder Abweichung ergibt dann Korrekturoffsetwerte für die Ortsberechnung. Diese Korrekturoffsetwerte werden in den Positionssollwerten berücksichtigt, wobei die absolute Position aller fünf Punkte miteinander verglichen wird.

Mit den auf dieser Weise kalibrierten Vieldrahtkammern wird anschließend die absolute Strahlposition überprüft, wobei eine Regelung derart durchgeführt wird, daß die auf diese Weise ermittelte Positionsabweichung maximal 25 % der Halbwertsbreite des Strahlprofils entspricht. Diese Interventionsschwelle relativ zur Halbwertsbreite des Strahlprofils erwies sich als praktikabel, da alle geometrischen Parameter eines Bestrahlungsplans mit der Halbwertsbreite skalieren und insbesondere die für den Patientenbetrieb erforderliche Qualität der erzeugten Teilchenbelegungen erreicht wird. Bei Durchführung einer Konstanzprüfung sollten lediglich die zuvor beschriebenen Vieldrahtkammermessungen eingesetzt werden, da die Installation eines zusätzlichen Profilgitters im Isozentrum für den täglichen Betrieb sehr aufwendig wäre.

Ein weiterer Aspekt dieses Prüfabschnitts umfaßt die Überwachung und Regelung der absoluten Strahlprofilbreite und der zeitlichen Stabilität. Die von der Beschleunigereinrichtung nach Anforderung durch eine Pulszentralenansteuerung des Kontrollsystems gelieferte Strahlfokussierung muß eingehalten werden, da auf diesen Werten die Behandlungs- bzw. Bestrahlungspläne beruhen. Zu diesem Zweck wird die absolute Strahlprofilbreite im Isozentrum 10 mit Hilfe eines Profilgitters überprüft, wobei die Lage des Profilgitters mit dem durch ein Laserkreuz auf einem Leuchttarget oder Film sichtbar gemachten Isozentrum justiert wird. Mit den Rasterscannermagneten 13, 14 wird der Behandlungsstrahl statisch in das Isozentrum abgelenkt, wobei dies beispielsweise bei etwa jedem zehnten Energieschritt erfolgen kann. Die mit der Profilgittermessung erhaltenen Strahlbreiten werden mit vorgegebenen Sollwerten verglichen, wobei eine Regelung derart erfolgt, daß eine maximale Abweichung der Strahlbreite von ±50 % von der Sollvorgabe eingehalten wird. Dies gilt insbesondere für den Energiebereich oberhalb 200 MeV/u.

Die Konstanzprüfung des Ionenstrahl-Therapiesystems kann wiederum mit den bereits zuvor beschriebenen Vieldrahtkammern erfolgen, die sich in einem Abstand von 970 mm bzw. 790 mm vor dem Isozentrum 10 befinden. Vor der eigentlichen Prüfung erfolgt eine Kalibrierung der absoluten Breitenmessung der beiden Vieldrahtkammern. Dabei wird ein Film mit horizontalen und vertikalen Streifen bestrahlt, wobei jeder Strahl mit einer Extraktion aus dem Synchrotron bei einer festen Fokussierung erzeugt wird. Auf diese Weise können abhängig von den auswählbaren Fokussierungen beispielsweise sieben Strahlen erzeugt werden. Die anhand des bestrahlten Films ermittelten Strahlbreiten werden mit den von den Vieldrahtkammern (Ortskammern) gemessenen verglichen, um daraus Korrekturoffsetwerte zu erhalten, welche anschließend wieder in den Sollwerten berücksichtigt werden können. Anschließend wird mit Hilfe der somit kalibrierten Vieldrahtkammern in Verbindung mit dem Kontroll. system die Halbwertsbreite des Strahlprofils sowie dessen zeitliche Konstanz oder Stabilität gemessen und überwacht, wobei dies insbesondere bei verschiedenen Energien und Intensitäten für die jeweils auswählbaren Fokussierungen durchgeführt wird.

Die zuvor beschriebene Anhebung der Interventionsschwelle von 20 % auf 50 % der Halbwertsbreite bei der Messung der absoluten Strahlprofilbreite im Vergleich zur Messung des absoluten Strahlorts ist kompatibel mit der Homogenitätsanforderung, da der Abstand der Strahlpositionen im Rahmen der Bestrahlungsplanung auf 33 % der Halbwertsbreite gesetzt wird.

Vor dem Isozentrum befinden sich üblicherweise einige Elemente zur Analyse und Modulation des Behandlungsstrahls, wie beispielsweise das Strahlaustrittsfenster, Detektoren oder ein Ripple-Filter. Durch diese Elemente wird eine Aufstreuung des Behandlungsstrahls hervorgerufen, welche mit abnehmender Strahlenergie stark zunimmt. Dadurch kann aus physikalischen Gründen eine Einhaltung der ursprünglich geforderten Strahlbreite im unteren Energiebereich (Energien < 200 MeV/u) nicht mehr oder nur schwer realisierbar sein. In diesem Fall wäre eine Überschreitung der oberen Toleranzwerte die Folge, so daß dieser Effekt bei der Bestrahlungsplanung berücksichtigt werden muß.

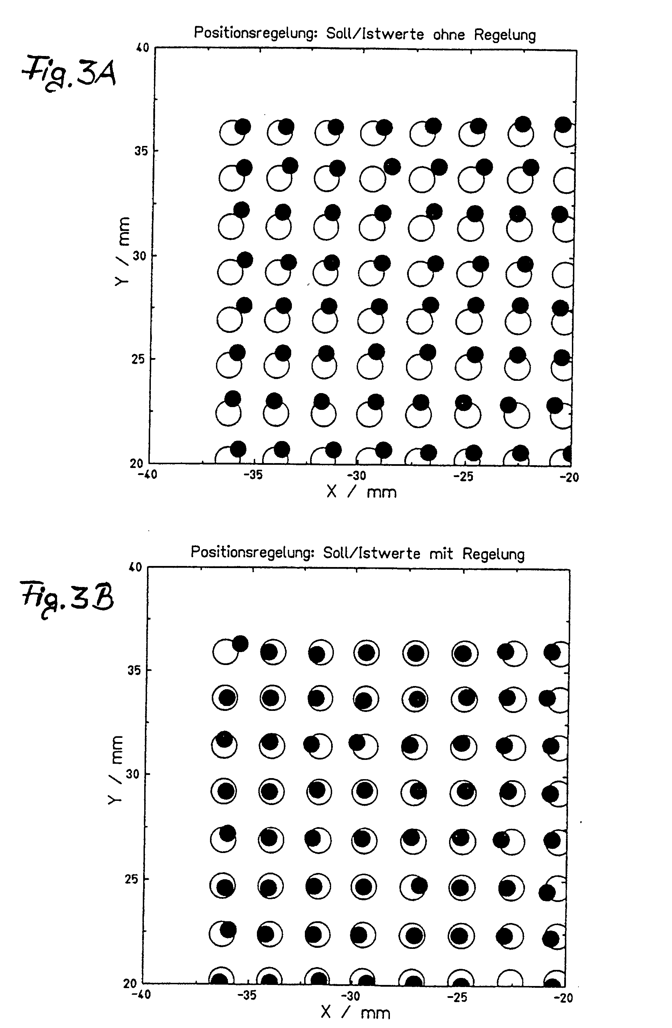

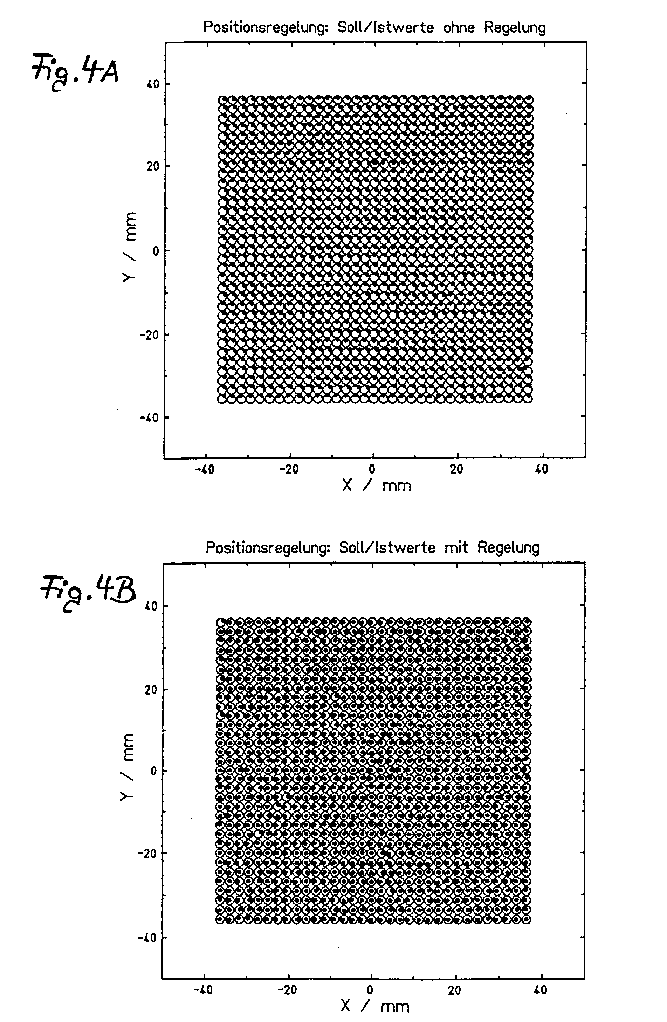

Die Auswirkungen der zuvor erläuterten Überwachungs- und Regelungsmaßnahmen bzgl. der Strahl-Nullpunktslage, des absoluten Strahlorts, der absoluten Strahlprofilbreite und deren zeitlichen Stabilität sind aus Fig. 3A/B und Fig. 4A/B ersichtlich. Die Fig. 3A und 3B entsprechen einer vergrößerten Darstellung der in Fig. 4A bzw. 4B gezeigten Diagramme. Dabei sind in Fig. 3A/4A Strahlpositionen ohne die zuvor vorgeschlagene Positionsregelung und in Fig. 3B/4B Strahlpositionen mit der Positionsregelung dargestellt. Den Darstellungen kann entnommen werden, daß durch Anwendung der Positionsregelung etc. eine deutlich stabilere Strahllage erzielt werden kann, während es ohne Positionsregelung teilweise zu starken Abweichungen aus der gewünschten Strahlposition kommt.

Ein weiterer Aspekt dieses Prüfabschnitts betrifft die Überwachung der Teilchenzahl im Behandlungsstrahl, d.h. die Überwachung der Variation der Teilchenzahl. Um den Meßbereich für Teilchenzahlmessungen nicht zu groß werden zu lassen, sollte die Intensität des vom Beschleuniger gelieferten Behandlungsstrahls lediglich innerhalb bestimmter Toleranzgrenzen schwanken. Im vorliegenden Fall wird vorgeschlagen, die Intensität des Behandlungsstrahls mit Hilfe der Ionisationskammern in Verbindung mit der Meßapparatur des Kontrollsystems zu messen und die Teilchenzahl über ein Zeitfenster von 300 µs zu mitteln. Die anschließend gemessenen Teilchenzahlen dürfen innerhalb des Zeitfensters maximal dem fünffachen Wert des zuvor erfaßten Mittelwerts entsprechen, um nicht eine Intervention auszulösen. Durch diese Maßnahmen kann ein sicherer Meßbereich gewählt werden, mit dem auch Teilchenzahlen noch korrekt gemessen werden können, die beispielsweise um einen Faktor 10 höher als der zuvor berechnete Mittelwert sind. Sollten noch höhere Teilchenzahlen auftreten, wird ein Alarm ausgelöst, und die bereits erwähnte Interlockeinheit löst die Strahlabschaltung aus. Es ist jedoch zu beachten, daß dieser Prüfaspekt lediglich die Voreinstellung der Detektoren betrifft und keinen direkten Einfluß auf die Energiedosis oder dergleichen hat. Auch bei einer Variation der Teilchenzahl, die deutlich oberhalb der zuvor definierten Interventionsschwelle liegt, kann die noch später beschriebene Homogenität der Teilchenbelegungen als entscheidendes Qualitätskriterium ausreichend sein.

Schließlich sollten hinsichtlich einer sicheren und stabilen Strahlführung am Bestrahlungsplatz auch die Sollpositionen sämtlicher beweglicher Teile zwischen den letzten Ablenkmagneten der Hochenergie-Strahlführungsleitung 6 und dem Drehgestell 8 regelmäßig überprüft werden, da jeder sich in der Strahlführung befindliche Gegenstand zu einer Beeinträchtigung der Strahlqualität am Bestrahlungsplatz führt. Es muß daher sichergestellt werden, daß sich keine beweglichen Teile der Strahlführung im Strahlweg befinden. Zu diesem Zweck sind an den entsprechenden beweglichen Teilen Endschalter angebracht, deren Zustände durch das Kontrollsystem automatisch und einzeln überprüft werden können. Dies sollte zur Konstanzprüfung vor jedem Bestrahlungsblock wiederholt werden.

Ein vierter Abschnitt des Prüfsystems betrifft die Überprüfung von Merkmalen, welche mit der Bestrahlungssteuereinheit des Ionenstrahl-Therapiesystems zusammenhängen.

Die in den zuvor beschriebenen Ionisationskammern des Überwachungs- oder Monitorsystems des Therapiesystems erzeugte elektrische Ladung, welche zur Bestimmung der Teilchenzahl dient, hängt vom Druck und der Temperatur des Ionisationskammergases ab, so daß diese beiden Größen während der Bestrahlung überwacht und protokolliert werden müssen. Der Druck und die Temperatur des Gases der Ionisationskammern werden mit Hilfe von elektrischen Sensoren gemessen, wobei die Meßwerte etwa einmal pro Minute vom Kontrollsystem erfaßt und mit eingetragenen Kalibrierfaktoren in absolute Einheiten (hPa und °C) umgerechnet und digital angezeigt werden. Der zeitliche Verlauf der Meßwerte kann in einem Trend-Diagramm grafisch dargestellt werden. Die Sensoren werden mit Hilfe von Referenzmeßgeräten kalibriert. Die Kalibrierung der in den Ionisationskammern angebrachten Sensoren sollte vor jedem Therapie-Bestrahlungsblock wiederholt werden. zusätzlich werden der Luftdruck und die Raumtemperatur am Ort des Überwachungssystems mit absolut kalibrierten Geräten gemessen und vom Kontrollsystem erfaßt sowie bei jeder Bestrahlung protokolliert. Somit können zur (täglichen) Überprüfung der Ionisationskammern die Absolutwerte für Luftdruck und Raumtemperatur direkt an den Referenzmeßgeräten abgelesen, mit den vom Kontrollsystem angezeigten Werten verglichen und protokolliert werden. Als Referenzwerte dienen dabei die bei der täglichen Kalibrierung des Überwachungssystems registrierten Meßwerte. Bei einer Abweichung von 20 hPa bzw. 5° C wird vom Kontrollsystem ein Alarm ausgelöst.

Auch das Laden von Programmen und Datensätzen in die Steuerungsrechner des Ionenstrahl-Therapiesystems muß überprüft werden. Dies ist erforderlich, um Daten, welche für eine Patientenbestrahlung erforderlich sind, korrekt in die Ablaufsteuerung des Systems laden zu können. Nur wenn alle Daten korrekt sind, darf eine Patientenbestrahlung aufgenommen werden. Zu diesem Zweck werden mit Hilfe von speziellen Programmen in den Serverrechnern des Kontrollsystems Programme und Daten in die einzelnen Prozessoren der Steuerungsrechner geschrieben, zurückgelesen und mit den in den eigenen Speichern gespeicherten Programmen und Daten verglichen, wobei diese Prüfprogramme vor jeder Bestrahlung automatisch ausgeführt werden. Nur wenn die zurückgeladenen Daten genau den in den Datenspeichern des Kontrollsystems gespeicherten Daten entsprechen, kann von einem sicheren Betrieb ausgegangen werden. Bei Abweichungen wird eine Alarmmeldung generiert und die zuvor beschriebene Interlockeinheit, welche zur Unterbindung einer Bestrahlung dient, kann nicht freigegeben werden.

Ein weiterer Prüfaspekt betrifft das Schalten der Ströme für die Ablenkmagnete 13, 14 des Rasterscanners. Dabei muß sichergestellt werden, daß die Stromwerte dieser Ablenkmagneten einen bestimmten, bei den Magnetnetzgeräten eingestellten Sollwert sowohl wertmäßig als auch zeitlich innerhalb bestimmter Toleranzgrenzen erreichen. Zu diesem Zweck wird die Zeit zwischen dem Setzen eines Magnetstromwerts in den Magnetnetzgeräten und dem Erreichen des entsprechenden stabilen Magnetstroms für verschiedene Stromwerte gemessen. Die dabei tolerierbare maximale Stromgenauigkeit bezüglich einer Abweichung vom eingestellten Magnetstromwert beträgt 0,3 A. Die maximal tolerierbare Einstellzeit bei einem Stromsprung von 2 A beträgt 175 µs in x-Richtung und 325 µs in y-Richtung. Werden diese Toleranzen nicht eingehalten, muß die Bestrahlung abgebrochen werden. Zur Konstanzprüfung kann dieser Test vor jedem Bestrahlungsblock durchgeführt werden.

Schließlich muß auch sichergestellt werden, daß die Nummer des bei Auftreten einer Abbruchbedingung aktiven Bestrahlungspunktes permanent, d.h. gegen Stromausfall gesichert, gespeichert wird. Dies ermöglicht eine von autorisierten Personen genehmigte Fortsetzung der Bestrahlung zu einem späteren Zeitpunkt. Die Funktionsfähigkeit dieser implementierten Sicherungsfunktion kann dadurch überprüft werden, daß ein bestimmter Bestrahlungs- oder Behandlungsplan in das Kontrollsystem geladen und ohne Bestrahlung ausgeführt, d.h. simuliert wird. Bei einem bestimmten Bestrahlungsort wird die Spannungsversorgung der Ablaufsteuerung ausgeschaltet und nach Wiederanfahren des Systems der letzte Bestrahlungsort ausgelesen und mit dem Bestrahlungsort bei Abschaltung der Spannungsversorgung verglichen. Bei Nichtübereinstimmung erfolgt ein entsprechender Eingriff. Diese Überprüfung wird zur Konstanzprüfung vor jedem Bestrahlungsblock durchgeführt.

Ein fünfter Abschnitt des Prüfsystems betrifft die Überprüfung der Funktionsfähigkeit der bereits zuvor beschriebenen Interlockeinheit des Ionenstrahl-Therapiesystems.

So müssen beispielsweise alle sicherheitsrelevanten Geräteparameter auf die Auslösung einer Notabschaltung des Systems bei Vorliegen eines Interlockfalls oder einer Interlockbedingung überprüft werden. Eine Abschaltung des Behandlungsstrahls 11 kann nur erfolgen, wenn ein Interlockfall erkannt wird. Daher müssen alle Quellen, die zu einem Interlockfall führen können einzeln in einem Test simuliert und die Auslösung des Interlocks, d.h. die Erzeugung der zur Notabschaltung des Behandlungsstrahls 11 führenden Signale durch die Interlockeinheit, überprüft werden. Die Interlockeinheit überwacht im Betrieb beispielsweise die Signale der oben beschrieben Endschalter der beweglichen Teile in der Strahlführung, die Zustände der Magnetnetzgeräte der Rasterscannermagneten 13 und 14, die Ionisationskammern hinsichtlich der Spannungsversorgung, eines Datenüberlaufs der Datenübertragung, der Einhaltung der Intensitätsgrenzwerte und des Gleichlaufs der einzelnen Ionisationskammern, die Elektronik der Strahlpositionsmeßeinrichtung sowie die Strahlposition selbst, die Hochspannung und den Gasfluß der einzelnen Detektoren, einen möglichen Interlock durch den Ablaufsteuerungsrechner, die Position der Patientenliege, eine mögliche Unterbrechung der Immobilisierung des Patienten (z.B. bei Öffnen der Maske am Bestrahlungsplatz oder bei Bewegung des Patienten), die Funktionsbereitschaft aller Rechnerprogramme und eine mögliche Notabschaltung oder Freigabe einer Bestrahlung durch die Medizinbedienkonsole des Therapiesystems etc.. Bei fehlender Auslösung des Interlocks bei Vorliegen eines Interlockzustands muß in das Therapiesystem eingegriffen und der Fehler behoben werden. Diese Überprüfung sollte zur Konstanzprüfung täglich durchgeführt werden.

Ebenso muß die Funktionsfähigkeit der manuellen Notabschaltung über die Medizinbedienkonsole überprüft werden, da eine manuelle Notabschaltung jederzeit gewährleistet sein muß.

Schließlich ist auch eine Überprüfung der Anzeigen aller sicherheitsrelevanten Zustände an den einzelnen Konsolen des Ionenstrahl-Therapiesystems, insbesondere der technischen Kontrollräume und des Hauptkontrollraums vonnöten. Die Anzeige dieser sicherheitsrelevanten Zustände dient einer schnellen Fehlererkennung sowie Fehlerbehebung und geben dem Betriebspersonal Informationen über den momentanen Stand der Bestrahlung. Diese Anzeigen der Alarmbedingungen können zusammen mit dem oben beschriebenen Test der Interlockeinheit überprüft werden. Zur Konstanzprüfung sollte dieser Test vor jedem Bestrahlungsblock und nach jeder Änderung des Kontrollsystems oder der Programme durchgeführt werden.

Ein sechster Abschnitt des Prüfsystems betrifft die Überprü.fung der medizinischen Einrichtungen zur Patientenpositionierung des Ionenstrahl-Therapiesystems.

So sollte beispielsweise die Genauigkeit der stereotaktischen Koordinatenbestimmung eines Zielpunkts mit Hilfe eines CToder MR-Verfahrens überprüft werden, da die Genauigkeit der stereotaktischen Bildgebung ein wesentlicher Faktor für die Gesamtgenauigkeit der Bestrahlung ist. Zu diesem Zweck kann ein beliebiger Zielpunkt innerhalb eines Kugelphantoms durch einen speziellen Prüfkörper repräsentiert werden, dessen Mittelpunkt gut sichtbar mit Hilfe der bildgebenden Verfahren darstellbar ist. Das Kugelphantom wird in den stereotaktischen Rahmen eingespannt, so daß der Mittelpunkt zu einem unbekannten Zielpunkt wird. Anschließend werden die stereotaktischen Koordinaten mit Hilfe der angewandten Röntgen-, CT- oder MR-Verfahren zeitlich nacheinander ermittelt, wobei bei den tomographischen Verfahren der Schichtabstand 1 mm betragen sollte. Da die Genauigkeit des Röntgenverfahrens auf 1/10 mm genau ist, kann die Genauigkeit der Zielpunktbestimmung mit CT und MR durch Vergleich mit dem Röntgenverfahren bestimmt werden, d.h. es wird der radiale Abstand zwischen der mit der Röntgenaufnahme ermittelten Position des Zielpunkts und der mit dem CT- bzw. MR-Verfahren ermittelten Position überprüft. Der radiale Abstand darf nicht mehr als 1,5 mm betragen. Zur Konstanzprüfung genügt die jährliche Durchführung dieses Tests.

Als weiterer Prüfaspekt wird vorgeschlagen, die Genauigkeit der Lage des Isozentrums zwischen der Drehachse der Patientenliege 9 und dem Zentralstrahl 11 des Rasterscanners 13, 14 zu überprüfen, da das als Schnittpunkt zwischen der Drehachse der Patientenliege 9 und dem Zentralstrahl 11 des Rasterscanners 13, 14 definierte Isozentrum das Verbindungselement bei der Positionierung zwischen Planung und Bestrahlung ist. Eine Konstanzprüfung sollte vor jedem Bestrahlungsblock durchgeführt werden.

Zur Überprüfung des Isozentrums in bezug auf die Drehachse der Patientenliege 9 wird ein metallischer Probekörper (2-3 mm Durchmesser) mit Hilfe von Lasern in das nominale Isozentrum, d.h. in die nominale Drehachse der Patientenliege 9, gebracht. Durch ein Senkblei, welches genau auf den Mittelpunkt oberhalb des Probekörpers gerichtet wird, wird die Position des Probekörpers festgehalten. Bei Rotation um die Drehachse der Patientenliege 9 wird das Ausmaß der Bewegung des Probekörpers in bezug auf das Senkblei ermittelt. Diese Prozedur wird in mindestens drei unterschiedlichen Höhen der Patientenliege 9, bei einer maximalen Verstellbarkeit der Patientenliege 9 um 15 cm nach oben/unten beispielsweise in Höhe des Isozentrum 10 sowie mit mindestens 15 cm Abstand nach oben und unten, durchgeführt. Die maximale Abweichung darf in Strahlrichtung 1,0 mm und senkrecht zur Strahlrichtung lediglich 0,5 mm betragen. Veränderungen in Strahlrichtung sind weniger kritisch, da Dosisverteilungen im Patienten nicht davon beeinflußt werden.

Zur Überprüfung des Isozentrums in bezug auf den Zentralstrahl 11 wird die Lage des Isozentrums per Definition auf der Drehachse der Patientenliege 9 unterhalb der Geradeaus-Strahlebene festgelegt und durch ein optisches Vermessungssystem in bezug auf Wandmarkierungen festgestellt. Die Prüfung der Position des Prüfkörpers in bezug auf den Zentralstrahl 11 erfolgt mit Hilfe einer Filmmessung, wobei ein Verifikationsfilm in Strahlrichtung gesehen hinter dem Probekörper mit einem (ungerasterten) Zentralstrahl bestrahlt wird, dessen Halbwertsbreite größer als der Durchmesser des Probekörpers ist, so daß sich die Position des Probekörpers auf dem Verifikationsfilm in bezug auf den Zentralstrahl abbildet. In diesem Fall liegt die Interventionsschwelle bei einer Abweichung von maximal 25 % der Halbwertsbreite des Primärstrahls.

Zudem muß die Genauigkeit der Laserjustierung auf das Isozentrum 10 überprüft werden, da die Laser das Isozentrum 10 markieren. Dabei werden die Laser nach Positionierung des Probekörpers in dem Isozentrum 10 mit Hilfe optischer Vermessung auf den Mittelpunkt des Probekörpers ausgerichtet und die Abweichung der Laserlinien von der Horizontalen bzw. Vertikalen überprüft, wobei die maximale Abweichung jeweils 1mm betragen darf. Für die Konstanzprüfung wird die Abbildung der Laser auf den gegenüberliegenden Wänden bzw. auf dem Fußboden markiert und dient anschließend als Bezugswert.

Ein weiterer Prüfaspekt betrifft die Genauigkeit der Justierung der Röntgenröhren und des Zielkreuzes auf den gegenüberliegenden Aufnahmestationen, da das Röntgenverfahren ein zusätzliches Verfahren zur Markierung des Isozentrums 10 darstellt. Nach Positionierung des Probekörpers in dem Isozentrum 10 mit Hilfe der optischen Vermessung, d.h. mit Hilfe der Laser, werden Röntgenaufnahmen in den drei Raumrichtungen durchgeführt und der Abstand der Abbildung zwischen dem Probekörper und dem Zielkreuz auf dem Röntgenbild ermittelt. Der Probekörper sollte sich exakt auf dem Bild des zielkreuzes abbilden, so daß der maximale Abstand zwischen der Abbildung des Probekörpers und dem Zielkreuz 1mm betragen darf.

Aufgrund der isozentrischen Bestrahlung der Patienten muß auch die Genauigkeit der Anzeige der Winkelskala der isozentrischen Rotation der Patientenliege 9 überprüft werden, wobei dies analog zu den Vorschriften der DIN 6847-5, Punkt 12.2.4 erfolgen kann. Die maximal tolerierbare Ungenauigkeit beträgt 1°.

Ebenso sollte die räumliche Stabilität der isozentrischen Rotation der Patientenliege 9 überprüft werden, da die Definition des Isozentrums 10 eine entsprechende Stabilität voraussetzt. Diese Überprüfung kann analog zu DIN 6847-5, Punkt 14.2 durchgeführt werden, wobei die Interventionsschwelle bei einer Ungenauigkeit von 1 mm liegt.

Schließlich wird auch vorgeschlagen, die Genauigkeit der Patientenlagerung und -positionierung zu überprüfen, da eine exakte Patientenpositionierung Voraussetzung für eine tumorkonforme Bestrahlung ist. Zur Abnahmeprüfung und Konstanzprüfung (vor jedem Bestrahlungsblock) des Therapiesystems können diesbezüglich die unbekannten stereotaktischen Koordinaten des Mittelpunkts eines Probekörpers, der innerhalb des stereotaktischen Grundrings fixiert wurde, als Zielpunkt ermittelt und der Mittelpunkt mit Hilfe des stereotaktischen Zielgeräts und durch transversale Bewegung der Patientenliege 9 in das Isozentrum 10 gebracht werden. In dieser Position werden in den drei Raumrichtungen Röntgenaufnahmen durchgeführt und der Abstand der Lage des Prüfkörpers vom Zielkreuz auf den drei Aufnahmen ermittelt. Der radiale Abstand zwischen dem Mittelpunkt des Prüfkörpers und dem Isozentrum darf maximal 1,5 mm betragen. Ansonsten ist eine entsprechende Korrektur der Patientenlagerung notwendig.

Ein siebter Abschnitt des Prüfsystems betrifft die Bestrahlungsplanung, in deren Verlauf insbesondere die für eine bestimmte Bestrahlung beabsichtigten Bestrahlungsdosiswerte berechnet werden.

Zunächst muß sichergestellt werden, daß für die Planung von Bestrahlungen, d.h. für die Berechnung jeder Bestrahlungsdosis, stets dieselben Basisdatensätze verwendet werden. Dies kann dadurch erfolgen, daß der Name, das Datum und die Größe der die Basisdaten enthaltenden Dateien mit den korrekten Bezeichnungen einer zuvor angelegten Sicherungskopie verglichen werden. Dies geschieht automatisch bei jedem Aufruf des Dosisberechnungsalgorithmus.

Auch die Identität der Werte der aktuellen Basisdatensätze mit den entsprechenden Werten einer Sicherungskopie muß überprüft werden, um sicherzustellen, daß die Basisdatensätze nicht unkontrolliert verändert worden sind. Auch hier erfolgt der Vergleich des Inhalts der aktuellen Basisdatensätze mit der Sicherungskopie mit Hilfe eines Computerprogramms, welches insbesondere vor jedem Bestrahlungsblock gestartet werden sollte.

Nach DIN 6873 Teil 5, Bestrahlungsplanungssysteme, ist zudem eine Prüfung der Bezugswerte im Basisdatensatz einmal im Monat erforderlich. Dieser Unterpunkt kann bei der vorliegenden Bestrahlungsplanung mit Schwerionen entfallen, da die Tiefendosisverteilungen, d.h. die Energieverlustdaten als Funktion der Tiefe, als absolute Werte in bezug auf die Eingangsfluenz abgespeichert sind. Somit ist kein besonderer Bezugswert für die Dosis aufgezeichnet. Die verwendeten Basisdatensätze werden bereits wie oben beschrieben überprüft.

Ein wesentlicher Aspekt bei der Prüfung der Bestrahlungsplanung ist die Überprüfung der Genauigkeit der in dem Ionenstrahl-Therapiesystem automatisch durchgeführten Dosisberechnung für eine geplante Bestrahlung in Abhängigkeit von den vorliegenden Basisdaten und den verwendeten Dosisberechnungsalgorithmen, wobei hier zwischen der Bestrahlung eines homogenen und eines inhomogenen Mediums zu unterscheiden ist. In beiden Fällen kann die Überprüfung der Dosisberechnung durch Verwendung eines Phantoms durchgeführt werden, was nachfolgend näher beschrieben wird.

Zur Überprüfung der berechneten Dosis für ein homogenes Medium können im Bestrahlungsplanungsprogramm des Ionenstrahl-Therapiesystems mehrere Meßpunkte, beispielsweise 10 Meßpunkte, in den berechneten Dosisverteilungen bzw. CT-Schnitten definiert werden, an denen die berechnete physikalische Dosis experimentell verifiziert werden soll. Die Verifikation erfolgt in einem Wasserphantom, wobei an den den gewünschten Meßpunkten entsprechenden Koordinaten im Wasserphantom Ionisationskammern positioniert werden. Das Bestrahlungsplanungsprogramm berechnet für die einzelnen Meßpunkte neben den Wasserenergiedosiswerten auch deren Koordinaten in dem verwendeten Phantom. Anschließend wird das Phantom mit den vom Bestrahlungsplanungsprogramm berechneten Steuerparametern bestrahlt, wobei die von dem Ionisationskammern erfaßten Werte in Energiedosiswerte umgerechnet werden, um die berechneten Dosiswerte zu verifizieren.

Die Verifikation wird für mehrere Bestrahlungspläne durchgeführt, wobei bevorzugt sechs typische Bestrahlungspläne verifiziert werden, von denen sich drei Pläne auf konstruierte Zielvolumina im Wasserphantom und drei Pläne auf die Bestrahlung von Patienten beziehen. Die letztgenannten Bestrahlungspläne werden nachfolgend als Standardpatientenpläne verwendet. Die von dem Bestrahlungsberechnungsprogramm berechneten Werte dienen als Bezugswerte für die durchzuführende Konstanzprüfung.

Als Interventionsschwelle wird festgelegt, daß die Abweichung zwischen den berechneten und den gemessenen Bestrahlungsdosiswerten insgesamt, d.h. im Mittel, maximal ±5 % der Dosis des Zielbestrahlungsvolumens betragen darf. Zudem wird festgelegt, daß die maximale Abweichung für einen einzelnen Meßpunkt ±7 % betragen darf.

Die oben beschriebene Vorgehensweise bezieht sich insbesondere für die Abnahmeprüfung des Ionenstrahl-Therapiesystems. Für eine Konstanzprüfung genügt die Verifikation von lediglich jeweils zwei der oben beschriebenen Standardpläne, um die Konstanz der berechneten Dosisverteilungen zu überprüfen und diese mit den experimentell zu bestimmenden Dosisverteilungen zu vergleichen. Die Konstanzprüfung sollte vor jedem Bestrahlungsblock durchgeführt werden.

Für die Überprüfung der Genauigkeit der Dosisberechnungen in Abhängigkeit von den Basisdaten, den verwendeten Bestrahlungsberechnungsalgorithmen und der verwendeten Näherung für ein inhomogenes Medium kann ein kugelförmiges Festkörperphantom verwendet werden, welches aus einem wasseräquivalenten Mate. rial besteht und aus einzelnen Schichten aufgebaut ist, in die zum Simulieren verschiedener inhomogener Körper unterschiedliche Inhomogenitäten eingesetzt werden können. Diese Inhomogenitäten sind Scheiben, die aus verschiedenen gewebeäquivalenten Materialien (beispielsweise entsprechend dem Material der Lunge, eines weichen oder harten Knochens, von Weichteilen oder von festem Wasser) bzw. lediglich Luft (bei Nichteinsetzen einer Scheibe) bestehen. Auch in diesem Fall werden in dem Phantom bis zu 10 Meßpunkte zur Verifikation definiert, an denen jeweils die Bestrahlungsdosis sowohl von dem Bestrahlungsplanungsprogramm berechnet als auch mit einem Satz von gleichzeitig messenden Ionisationskammern erfaßt und damit verglichen wird.

Es wird vorgeschlagen, zur Abnahmeprüfung drei unterschiedliche Phantomaufbauten zur Untersuchung der berechneten Dosisverteilung hinter Grenzschichten verschiedener Materialien (beispielsweise Luft/Wasser und Knochen/Wasser), in dünnen Inhomogenitäten und in dicken Inhomogenitäten durchzuführen.

Auch bei der Untersuchung der berechneten Dosiswerte für inhomogene Medien wird als Toleranzschwelle vorgeschlagen, eine maximale mittlere Abweichung zwischen den berechneten Dosiswerten und den gemessenen Dosiswerten aller Meßpunkte von ±5 % und eine maximale Abweichung für einen einzelnen Meßpunkt von ±7 % zuzulassen. Zur Konstanzprüfung können die zuvor beschriebenen Tests vor jedem Bestrahlungsblock durchgeführt werden.

Ebenso können die Dosisberechnungen durch Verwendung eines irregulär geformten Prüfphantoms verifiziert werden. In diesem Fall wird ein Prüfphantom benutzt, welches aus wasseräquivalentem Material besteht und beispielsweise einen menschlichen Kopf nachbildet. Wie zuvor beschrieben, werden in dem Phantom bis zu 10 Meßpunkte zur Verifikation definiert. Des weiteren werden Bestrahlungsparameter für ein geeignetes Zielbestrahlungsvolumen in dem Kopfphantom festgelegt und das Prüfphantom mit Hilfe des stereotaktischen Grundrings justiert. Anschließend wird die von dem Bestrahlungsplanungsprogramm des Ionen-Therapiesystems berechneten Werte der Wasserenergiedosis an den gewählten Meßorten anhand der mit den Ionisationskammern an diesen Meßpunkten gemessenen Werten verglichen, wobei wiederum die Abweichung für sämtliche Meßpunkte maximal ±5 % der Dosis des Zielbestrahlungsvolumens betragen darf, während für jeden einzelnen Meßpunkt eine maximale Abweichung von ±7 % zulässig ist. Zur Konstanzprüfung kann dieser Test vor jedem Bestrahlungsblock durchgeführt werden.

Ein weiterer Aspekt bei der Überprüfung der Bestrahlungsplanung betrifft die Prüfung der in dem Ionenstrahl-Therapiesystem eingesetzten bildgebenden Verfahren, um eine korrekte Übertragung der geometrischen Strukturen (z.B. des Zielbestrahlungsvolumens und der Konturen des Patienten) sowie der Planungsparameter von der Bildgebung bis zur Positionierung sicherzustellen. Zu diesem Zweck kann wie bei der Verifikation der berechneten Bestrahlungsdosiswerte im inhomogenen Medium ein Phantom mit scheiben- oder ringförmigen Einsätzen verwendet werden, wobei in diesem Fall die inhomogenen Einsätze zudem unterschiedliche Durchmesser aufweisen können. Von dem Phantom wird ein Bild aufgenommen, und es werden aus den somit erhaltenen CT-Daten für die drei Hauptrichtungen in dem Drehgestell 8 (vgl. Fig. 2) digitale Röntgen-Rekonstruktionen berechnet. Anschließend wird eine Verifikation der Planungsgeometrie mit Hilfe von Röntgenaufnahmen des Röntgenpositioniersystems in den drei Hauptrichtungen durchgeführt. Diese Vorgehensweise kann unter verschiedenen Winkeln der in Fig. 2 gezeigten Patientenliege 9 durchgeführt werden, z.B. unter 0°, 45° und 90°. Auf diese Weise wird die Form und die Lage der Inhomogenitäten in der digitalen Röntgen-Rekonstruktion in bezug auf die Röntgenaufnahmen des Röntgenpositioniersystems verifiziert. Als Toleranzschwellen wird in diesem Fall festgelegt, daß sowohl die maximale positionale Abweichung als auch die maximale Abweichung hinsichtlich der Form der Ringe des Phantoms 2 mm betragen darf. Die Konstanzprüfung kann wiederum vor jedem Bestrahlungsblock durchgeführt werden.

Zur Erhöhung der Betriebssicherheit ist zudem eine Überwachung der Wartung und Weiterentwicklung der in dem Ionenstrahl-Therapiesystem eingesetzten Bestrahlungsplanungsprogramme erforderlich. Nach einer Weiterentwicklung der Bestrahlungsplanungsprogramme kann versehentlich eine falsche Version verwendet werden. Um dies zu vermeiden und sicherzustellen, daß stets die korrekten Versionen der unterschiedlichen Module verwendet werden, wird das Kontrollsystem des Ionenstrahl-Therapiesystems derart ausgestaltet, daß bei jedem Aufruf eines Bestrahlungsplanungsprogramms Versionsnummern mit Datum des jeweiligen Programms angezeigt werden, die von dem Benutzer mit Daten in einem Protokollbuch zu vergleichen sind.

Ebenso muß sichergestellt werden, daß bei einer Weiterentwicklung des Bestrahlungsplanungsprogramms, d.h. bei Vorliegen einer neuen Version, diese nur nach einer erneuten Abnahmeprüfung wirksam wird. Dies kann dadurch geschehen, daß komplette Dosisverteilungen wie zuvor beschrieben für ein homogenes Medium, ein inhomogenes Medium und für ein irregulär geformtes Phantom berechnet und als Sicherungskopie gespeichert werden. Bei Verwendung der neuen Programmversion können diese gespeicherten Dosiswerte als Bezugs- oder Referenzwerte zur Verifikation der Funktionsfähigkeit der neuen Programmversion verwendet werden, da auch bei Verwendung der neuen Programmversion für dasselbe Phantom dieselben Dosiswerte berechnet werden müßten. Diese Überprüfung sollte daher nach jeder Änderung eines Bestrahlungsplanungsprogramms durchgeführt werden.

Ein achter Abschnitt des Prüfsystems betrifft die Überprüfung des Rasterscanvorgangs sowie der Dosimetrie.

Dabei betrifft ein erster Prüfaspekt dieses Prüfabschnitts die Teilchenzahlmonitore- bzw. -überwachungsmittel des Ionenstrahl-Therapiesystems, welche bei dem vorliegenden Ausführungsbeispiel - wie bereits beschrieben worden ist - aus großflächigen Ionisationskammern bestehen.

Diesbezüglich muß beispielsweise die Konstanz der Kalibrierfaktoren dieser Ionisationskammern überprüft werden, da sich die Kalibrierfaktoren nur im Rahmen von Luftdichteschwankungen ändern dürfen. Die beiden Ionisationskammern des Rasterscanners sind hinsichtlich der Teilchenzahl pro Überwachungs- oder Monitoreinheit der Ionisationskammern kalibriert. Die Kalibrierung wird durch einen Kalibrierfaktor K beschrieben, der von der Bestrahlungsenergie E der Teilchen und der Schrittweite $\Delta x$ und $\Delta y$ des Rasterscanners abhängt, d.h. $K = K(E, \Delta x, \Delta y)$. Die Kalibrierung der Ionisationskammern erfolgt über eine Dosismessung in einem homogen gerasterten Bestrahlungsfeld, wobei die Abweichungen von den Bezugsbedingungen korrigiert werden und die Anzeige der Ionisationskammer in eine Wasserenergiedosis $D_{scan}$ umgerechnet wird. Der Kalibrierfaktor wird berechnet nach:

$$K(E, \Delta x, \Delta y) = (D_{scan}/M_i) \cdot \Delta x \cdot \Delta y / (S(E)/\rho)$$

mit $(S(E)/\rho)$ = Massenbremsvermögen von $^{12}C$ bei einer Bestrahlungsenergie E, und

M = Monitoreinheiten pro Koordinatenpunkt i der Ionisationskammer.

Der relevante Energiebereich (beispielsweise zwischen 80 MeV/u und 430 MeV/u) wird in mehreren Schritten vermessen. Der Meßort der jeweils überprüften Ionisationskammer befindet sich im Isozentrum 10, wobei die Ionisationskammer bzw. das Dosimeter in einem Festkörperphantom angeordnet ist. Es wird dieselbe Tabelle des Massenbremsvermögens von $^{12}C$ verwendet, die auch der Bestrahlungsplanung zugrunde gelegt wird. Auf diese Weise wird in Abhängigkeit von der Energie E und der Schrittweite $\Delta x, \Delta y$ ein Satz von Kalibrierfaktoren K erhalten, wobei festgelegt wird, daß die Abweichung von den Bezugswerten maximal ±3 % für jeden Kalibrierfaktor betragen darf. Aus dem Satz von Kalibrierfaktoren sollten mindestens drei Werte überprüft werden. Zur Konstanzprüfung sollte dieses Prüfverfahren täglich durchgeführt werden.

Auch die Dosiskonstanz muß überprüft werden, da gleiche vorgewählte Monitoreinheiten der Ionisationskammern stets zu gleichen Dosisanzeigen führen müssen. Es empfiehlt sich daher, die Konstanz der Dosis im Mittelpunkt von würfelförmigen Bestrahlungsvolumina, die von dem Rasterscanner bzw. dessen Magneten 13, 14 erzeugt oder abgetastet werden, in Abhängigkeit von dem Satz der Kalibrierfaktoren der Ionisationskammern zu überprüfen. Zu diesem Zweck wird zum Erhalt von Referenzwerten die Dosis in einem Phantom gemessen, welches derart positioniert ist, daß sich das Isozentrum 10 genau in der Mitte seiner vorderen Oberfläche befindet. Die Bestrahlung erfolgt dabei innerhalb eines Bestrahlungs- oder Dosiswürfels mit 5 cm Kantenlänge, dessen Mittelpunkt als Meßort in 11,3 cm wasseräquivalenter Tiefe angeordnet ist. (Die Berechnung der Steuerdaten zur Erzeugung des Dosiswürfels erfolgt mit Hilfe der CT-basierten Bestrahlungsplanung. Für diesen Schritt ist es zweckmäßiger, das Isozentrum 10 auf den Ort des Strahleintritts in das Wasserphantom zu legen. Des weiteren gestattet die gewählte Meßtiefe eine Vereinheitlichung der Meßausrüstung für die unterschiedlichen Tests.) Die auf diese Weise bestimmte Bestrahlungsdosis wird als Referenzdosis gespeichert. Die nachfolgend gemessenen tatsächlichen Dosiswerte können dann mit dieser Referenzdosis verglichen werden, wobei eine maximale Abweichung zwischen der tatsächlichen und der nominalen Dosis (Referenzdosis) von ±3 % zulässig ist. Dabei sollte eine tägliche Konstanzprüfung durchgeführt werden.

Auch die Einflußparameter auf die Teilchenzahlmonitore bzw. Ionisationskammern müssen überprüft werden, wobei dabei insbesondere die Abhängigkeit der Kalibrierfaktoren K von der Teilchenfluenz und dem Teilchenfluß überprüft wird. In beiden Fällen sollte eine jährliche Konstanzprüfung durchgeführt werden.

Zur Überprüfung der Abhängigkeit der Kalibrierfaktoren von der Teilchenfluenz wird im wesentlichen dasselbe Verfahren wie bei der Überprüfung der Konstanz der Kalibrierfaktoren durchgeführt. Die Messungen werden in einem Phantom durchgeführt, welches mit einer Fläche von 5 x 5 cm$^2$ bei den Energien 150 MeV/u, 250 MeV/u und 350 MeV/u mit jeweils gleicher Strahlintensität bestrahlt wird. Eine Ionisationskammer wird in der Mitte der bestrahlten Fläche angeordnet, Die Monitorwerte der Ionisationskammer werden so festgelegt, daß sich am Meßort eine Dosis von 0,2 Gy, 0,5 Gy bzw. 1 Gy ergibt. Für diese unterschiedlichen Monitorwerte wird die Übereinstimmung zwischen der tatsächlichen und der nominalen Dosis erfaßt, wobei eine maximale Abweichung von ±3 % zulässig ist. Die Einhaltung dieser engen Toleranz ist sinnvoll und auch durchführbar.

Zur Überprüfung der Abhängigkeit der Kalibrierfaktoren von dem Teilchenfluß wird ebenfalls im wesentlichen dasselbe Verfahren wie bei der Überprüfung der Konstanz der Kalibrierfaktoren angewendet. In diesem Fall wird jedoch die Dosis konstant gehalten und die Strahlintensität jeweils auf einen hohen, mittleren und niedrigen Wert eingestellt, so daß die Übereinstimmung der tatsächlichen Bestrahlungsdosis mit der nominalen Referenzdosis für unterschiedliche Intensitäten überprüft werden kann. Auch hier ist eine maximale Abweichung von ±3 % zulässig.

Hinsichtlich der Ionisationskammern bzw. Teilchenzahlmonitore sollte auch die Abhängigkeit deren Kalibrierfaktoren von der Strahllage überprüft werden. Es wird im wesentlichen dasselbe Verfahren wie bei der Überprüfung der Konstanz der Kalibrierfaktoren durchgeführt, wobei jedoch dieselbe Anordnung wie bei der zuvor beschriebenen Überprüfung der Dosiskonstanz verwendet wird. Die Messungen werden in einem Bestrahlungsvolumen bzw. Bestrahlungswürfel des Rasterscanners 13, 14 mit einer Kantenlänge von 5 cm durchgeführt, jedoch mit einer seitlichen Versetzung von 2 cm und 6 cm. Dabei werden die Monitorwerte der Ionisationskammern derart festgelegt, daß sich in der Mitte des Bestrahlungsvolumens eine Bestrahlungsdosis von 1 Gy ergibt. Bei der Überprüfung der Anzeigen der Ionisationskammern sollte sich der seitlich gemessene Wert nicht mehr als 3 % von dem in der Mitte gemessenen Wert unterscheiden. Auch in diesem Fall wird eine jährliche Konstanzprüfung empfohlen.

Ein weiterer Prüfaspekt dieses Prüfabschnitts betrifft die Überprüfung der Dosisverteilung des Rasterscanners 13, 14, wobei sowohl die Tiefendosisverteilung'als auch die Dosisquerverteilung überprüft wird.

Die Homogenität der Tiefendosisverteilung wird in Abhängigkeit von einer ausgewählten Bestrahlungsenergie und ausgewählten Monitorwerten pro Bestrahlungsenergiewert der verwendeten Ionisationskammern überprüft, da die Tiefendosishomogenität entscheidend von der gewählten Energie und deren Konstanz abhängt. Zu diesem Zweck werden mit den Rasterscannermagneten 13, 14 in einem Phantom erneut quader- oder würfelförmige Bestrahlungsvolumina erzeugt, wobei für jeden Koordinatenpunkt einer Schicht (Energie) eine konstante Teilchenbelegung, jedoch pro Schicht eine unterschiedliche Teilchenbelegung derart verwendet wird, daß sich in dem Bestrahlungswürfel eine homogene Dosisverteilung ergibt. Mehrere Dosimeter (Ionisationskammern), beispielsweise 10 Ionisationskammern, messen in unterschiedlichen wasseräquivalenten Tiefen, wobei die Ionisationskammern derart positioniert werden, daß nicht mehrere Ionisationskammern hintereinander bestrahlt werden. Die Kantenlängen der Bestrahlungswürfel betragen beispielsweise 2,5 cm, 5 cm und 10 cm, wobei die Messungen der Ionisationskammern für Tiefen der Mittelpunkte der jeweiligen würfelförmigen Bestrahlungsvolumens von 5 cm, 12,5 cm bzw. 20 cm durchgeführt werden. Die Monitorwerte werden aus der Bestrahlungsplanung in der Weise festgelegt, daß sich in der Mitte des jeweiligen Bestrahlungsvolumens eine durch die Bestrahlungsplanung vorgegebene Bestrahlungsdosis ergibt. Durch Vergleich der tatsächlichen Meßwerte mit den Referenzwerte kann die Schwankungsbreite der Anzeigen der Ionisationskammern überprüft werden. Eine maximale Abweichung von ±5 % ist tolerierbar. Bei Überschreiten dieser Toleranzgrenze muß in das System eingegriffen werden, um die zu große Abweichung zu korrigieren. Zur Konstanzprüfung sollte das oben beschriebene Prüfverfahren vor jedem Bestrahlungsblock durchgeführt werden.

Die Dosisquerverteilung des Rasterscanners wird in Abhängigkeit von der Energie überprüft, um sicherzustellen, daß die Homogenität des Rasterscanverfahrens bei allen verwendeten Bestrahlungsenergien gewährleistet ist. In diesem Fall wird bei festen Ionisationskammer-Monitorwerten und jeweils unterschiedlichen Bestrahlungsenergien (z.B. 100 MeV/u, 150 MeV/u, 200 MeV/u, 250 MeV/u, 300 MeV/u und 350 MeV/u) und Strahlfeldern die Bestrahlungsdosis senkrecht zur Strahlrichtung mit mehreren gleichzeitig messenden Ionisationskammern festgestellt. Gleichzeitig wird vor den Dosimetern bzw. Ionisationskammern frei Luft eine Schwärzungsverteilung auf einem Verifikationsfilm erzeugt. Mit dem Rasterscanner 13, 14 werden Flächen mit einer Seitenfläche von beispielsweise 5 cm, 10 cm und 18 cm erzeugt, wobei die Bestrahlungsdosis jeweils ca. 1 Gy betragen soll. Es wird die Standardabweichung der korrigierten Anzeigen der Ionisationskammern bzw. der Verifikationsfilmschwärzung innerhalb des Bestrahlungsfeldes überprüft, wobei eine maximale Abweichung von den Bezugswerten von ±5 % tolerierbar ist. Nicht tolerierbare Abweichungen von den Bezugswerten werden korrigiert, um eine Anpassung an die tatsächlich vorliegenden Meßbedingungen zu erzielen. Eine Konstanzprüfung sollte vor jedem Bestrahlungsblock durchgeführt werden, wobei in diesem Fall die Verwendung des Verifikationsfilms mit Überwachung der Schwärzung dieses Verifikationsfilms genügt.

Ein weiterer Prüfaspekt dieses Prüfabschnitts betrifft die Überprüfung der Feldgeometrie beim Rasterscanverfahren, wobei die Abhängigkeit der räumlichen Lage eines bestimmten Bestrahlungsvolumens des Rasterscanners 13, 14 von gewählten Bestrahlungsenergien überprüft wird. Zu diesem Zweck werden von dem Rasterscanner 13, 14 würfel- oder quaderförmige Bestrahlungsvolumina erzeugt, wobei eine konstante Teilchenbelegung für jeden Koordinatenpunkt einer Schicht (Energie), jedoch eine unterschiedliche Belegung pro Schicht derart verwendet wird, daß sich eine homogene Dosisverteilung in dem Bestrahlungswürfel ergibt. Unter diesen Bedingungen wird ein keilförmiges Festkörperphantom bestrahlt, hinter dem sich ein Verifikationsfilm befindet. Anschließend wird die Position der Verifikationsfilmschwärzung relativ zu dem Mittelpunkt der Bestrahlung festgestellt.

Bei der Messung betragen die Kantenlängen der Bestrahlungsfelder beispielsweise 4 cm, 7 cm und 12 cm, während die Ausdehnung der Bestrahlungsquader oder -würfel in Strahlrichtung 2,5 cm, 5 cm und 10 cm betragen. Die Messungen werden dabei für wasseräquivalente Tiefen des Mittelpunkts der jeweiligen Bestrahlungsvolumina von 5 cm, 12,5 cm bzw. 20 cm durchgeführt. Die Monitorwerte der Dosimeter bzw. Ionisationskammern werden derart aus der Bestrahlungsplanung festgelegt, daß sich in der Mitte des Bestrahlungsvolumens eine durch die Bestrahlungsplanung vorgegeben Bestrahlungsdosis ergibt. Als Feldgrenzen werden die Orte definiert, an denen der Randabfall der Schwärzung bei 50 % des Plateauwerts liegt. Die Lage der distalen Feldgrenzen sowie der in Strahlrichtung gesehen seitlichen Feldgrenzen werden überprüft und mit Bezugswerten verglichen. Eine Abweichung von 2 mm in jeder Richtung ist tolerierbar, ansonsten muß eine Korrektur des Systems erfolgen, um das System an die tatsächlich vorliegenden Meßbedingungen anzupassen. Dieses Prüfverfahren sollte zur Konstanzprüfung vor jedem Bestrahlungsblock durchgeführt werden, wobei hier eine Auswahl von jeweils drei Bedingungen aus den Kombinationen der zuvor beschriebenen Bedingungen genügt.

Schließlich betrifft ein weiterer Prüfaspekt dieses Prüfabschnitts die Verifikation des Gesamtsystems, um die Genauigkeit der applizierten Bestrahlungsdosis hinsichtlich ihrer Höhe und Räumlichkeit bei jedem zu bestrahlenden Patienten verifizieren zu können, so daß eine korrekte Zusammenarbeit der einzelnen Komponenten des Systems gewährleistet wird. Dabei wird zwischen der Bestrahlung eines homogenen Mediums und eines inhomogenen Mediums unterschieden.

Im ersten Fall wird wie bei der zuvor beschriebenen Verifikation der Übereinstimmung von berechneten und gemessenen Dosisverteilungen für ein homogenes Medium ein homogenes Phantom verwendet und im wesentlichen dasselbe Verfahren durchgeführt, jedoch mit der Ausnahme, daß nunmehr individuelle Patientenbestrahlungspläne als Grundlage dienen. Für sämtliche Meßpunkte wird der Unterschied zwischen der berechneten Bestrahlungsdosis und der gemessenen Bestrahlungsdosis ermittelt, wobei wiederum eine mittlere Abweichung für sämtliche Meßpunkte von 5 % und eine Abweichung für einen einzelnen Meßpunkt von 7 % tolerierbar ist. Zur Konstanzprüfung sollte dieser Test vor jedem Bestrahlungsblock durchgeführt werden.

Zur Überprüfung der Genauigkeit bei Bestrahlung eines zu bestrahlenden inhomogenen Mediums wird wiederum ein inhomogenes Phantom verwendet, wobei in diesem Fall als einmalige Vorbereitung für ein halbkugelförmiges Phantom aus einem festen, wasseräquivalentem Material mit einem Radius von beispielsweise 8cm eine Bestrahlungsplanung durchgeführt wird. Für die Bestrahlungsplanung befindet sich der Mittelpunkt des Phantoms im Isozentrum 10 und die Halbkugel des Phantoms ist der Einstrahlrichtung entgegengewandt. In das Phantom können unterschiedliche Inhomogenitäten, beispielsweise in Form von Scheiben mit einem Durchmesser von jeweils 3 cm, eingelegt werden, wobei bevorzugt sieben verschiedene Materialien oder Inhomogenitäten mit folgenden Dichten verwendet werden:

| Nr. | Dichte | |
|-----|--------|----------------|
| 1 | 0,001 | (Luft) |
| 2 | 0,30 | (Lunge) |
| 3 | 1,035 | (festes Wasser) |
| 4 | 0,92 | (Fett) |
| 5 | 1,05 | (Muskel) |
| 6 | 1,14 | (weicher Knochen) |
| 7 | 1,84 | (harter Knochen) |

Das geplante Zielbestrahlungsvolumen ist für drei unterschiedliche Einstrahlrichtungen mit einem Einstrahlwinkel von 0°, +45° und -45° jeweils eine 2 cm dicke Schicht innerhalb des Halbkugelphantoms, die unmittelbar an die flache Seite der Halbkugel angrenzt, so daß die distale Lage des Bestrahlungsvolumens mit der hinteren Flachseite übereinstimmt. Die im Zielbestrahlungsvolumen geplante homogene Bestrahlungsdosis beträgt 1 Gy. Mit diesen Steuerdaten für die Steuerung des Rasterscanners werden die Bestrahlungen mit den drei Einstrahlrichtungen durchgeführt, wobei sowohl im Zielbestrahlungsvolumen als auch hinter jeder Inhomogenität ein Dosimeter (d.h. eine Ionisationskammer) positioniert ist, deren Anzeige überwacht wird. Die ermittelte Energiedosis in allen Meßpunkten sollte innerhalb des Zielbestrahlungsvolumens nicht die Schwelle 1 Gy $\pm 5$ % überschreiten, während 5 cm hinter dem Zielbestrahlungsvolumen eine maximale Abweichung von $\pm 10$ % von der berechneten und auf das Zielbestrahlungsvolumen bezogenen Bestrahlungsdosis tolerierbar ist. Zudem ist für sämtliche Meßpunkte erneut eine mittlere Abweichung der gemessenen Bestrahlungsdosis von $\pm 5$ % und für jeden einzelnen Meßpunkt eine maximale Abweichung von $\pm 7$ % tolerierbar. Zur Konstanzprüfung sollte dieses Prüfverfahren vor jedem Bestrahlungsblock durchgeführt werden.

## Patentansprüche

1. Verfahren zum Betreiben eines Schwerionenstrahl-Therapiesystems unter Überwachung der Bestrahlungsdosisverteilung,
wobei das Schwerionenstrahl-Therapiesystem umfaßt

   - mindestens eine Ionenquelle (1),
   - eine Beschleunigereinrichtung (2, 5) zum Beschleunigen der Ionen der Ionenquelle (1) in Form eines Schwerionenstrahls (11),
   - ein Strahlführungssystem (6, 8), um den Schwerionenstrahl (11) von der Beschleunigereinrichtung (2, 5) mindestens einem Bestrahlungsplatz zur Behandlung eines Patienten zuzuführen, wobei das Strahlführungssystem (6, 8) mindestens eine Strahlführungsleitung (6) umfaßt, und
   - eine in dem Strahlführungssystem (6, 8) angeordnete Rasterscannereinrichtung mit vertikalen Ablenkmitteln (13) und horizontalen Ablenkmitteln (14) zur vertikalen bzw. horizontalen Ablenkung des Schwerionenstrahls (11) senkrecht zu seiner Strahlrichtung, so daß der Schwerionenstrahl (11) von der Rasterscannereinrichtung auf ein Isozentrum (10) des Bestrahlungsplatzes abgelenkt wird und eine bestimmte, das Isozentrum (10) umgebende Fläche mit einer bestimmten Bestrahlungsdosis abtastet,

   **dadurch gekennzeichnet,**
   **daß** die Bestrahlungsdosisverteilung der Rasterscannereinrichtung (13, 14) an verschiedenen Positionen im Bereich des Isozentrums (10) gemessen und ausgewertet wird, und
   **daß** auf eine ausreichend homogene Bestrahlungsdosisverteilung geschlossen wird, falls die Schwankungsbreite der an den einzelnen Positionen gemessenen Bestrahlungsdosiswerten einen bestimmten Toleranzgrenzwert nicht überschreitet, und daß Überwachungsmittel in Übereinstimmung mit einem Kalibrierfaktor K hinsichtlich der von ihnen erfaßten Teilchenzahl pro Überwachungseinheit der Überwachungsmittel kalibriert werden, wobei der Kalibrierfaktor K als Funktion der Bestrahlungsenergie E der Teilchen des Schwerionenstrahls (11) und der Abtastschrittweite $\Delta x$, $\Delta y$ der Rasterscannereinrichtung (13, 14) berechnet wird durch:

   $$K(E, \Delta x, \Delta y) = (D_{scan}/M_i) \cdot \Delta x \cdot \Delta y / (S(E)/\rho),$$

wobei der Ausdruck S(E)/ρ dem Massenbremsvermögen der Teilchen des Schwerionenstrahls (11) bei der Bestrahlungsenergie E entspricht,

wobei M der Anzahl der Überwachungseinheiten pro Koordinatenpunkt i der Überwachungsmittel entspricht, und

wobei $D_{scan}$ dem von den Überwachungsmitteln erfaßten Bestrahlungsdosiswert, umgerechnet in einen Wasser-Energiedosiswert, entspricht.

2. Verfahren nach Anspruch 1,
   **dadurch gekennzeichnet,**
   **daß** die Bestrahlungsdosisverteilung der Rasterscannereinrichtung (13, 14) in Strahlrichtung des Schwerionenstrahls (11) gemessen und ausgewertet wird.

3. Verfahren nach Anspruch 2,
   **dadurch gekennzeichnet,**
   **daß** eine Bestrahlung eines Phantoms mit der Rasterscannereinrichtung (13, 14) durch Erzeugung von Bestrahlungsvolumina bestimmter Form durchgeführt wird, wobei die Bestrahlung derart erfolgt, daß sich in jedem Bestrahlungsvolumen eine homogene Dosisverteilung einstellt, und
   **daß** die Bestrahlungsdosis an unterschiedlichen Tiefenpositionen des Phantoms gemessen und mit einem Referenzwert verglichen wird.

4. Verfahren nach Anspruch 3,
   **dadurch gekennzeichnet,**
   **daß** die Form der von der Rasterscannereinrichtung (13, 14) erzeugten Bestrahlungsvolumina quaderförmig ist.

5. Verfahren nach Anspruch 4,
   **dadurch gekennzeichnet,**
   **daß** die Bestrahlung durch die Rasterscannereinrichtung (13, 14) derart erfolgt, daß die Kantenlängen der quaderförmigen Bestrahlungsvolumina in etwa 2,5 cm, 5 cm und 10 cm betragen.

6. Verfahren nach einem der Ansprüche 3 bis 5,
   **dadurch gekennzeichnet,**
   **daß** die Bestrahlungsdosis an den unterschiedlichen Tiefenpositionen des Phantoms mit Ionisationskammern gemessen werden.

7. Verfahren nach einem der Ansprüche 3 bis 6,
   **dadurch gekennzeichnet,**
   **daß** die unterschiedlichen Tiefenpositionen, an denen die Bestrahlungsdosis gemessen wird, einer Tiefe des quaderförmigen Bestrahlungsvolumens von 5 cm, 12,5 cm bzw. 20 cm entsprechen.

8. Verfahren nach einem der vorhergehenden Ansprüche,
   **dadurch gekennzeichnet,**

**daß** die Bestrahlungsdosisverteilung der Rasterscannereinrichtung (13, 14) quer zur Strahlrichtung des Schwerionenstrahls (11) gemessen und ausgewertet wird.

9. Verfahren nach Anspruch 8,
   **dadurch gekennzeichnet,**
   **daß** ein Phantom mit der Rasterscannereinrichtung (13, 14) durch Erzeugung von Bestrahlungsflächen bestrahlt wird, wobei mehrere Bestrahlungen mit unterschiedlichen Bestrahlungsenergien und unterschiedlichen Bestrahlungsflächen durchgeführt und die Bestrahlungsdosis jeweils senkrecht zur Strahlrichtung des Schwerionenstrahls (11) gemessen wird.

10. verfahren nach Anspruch 9,
    **dadurch gekennzeichnet,**
    **daß** die unterschiedlichen Bestrahlungsenergien der mehreren Bestrahlungen 100 MeV/u, 150 MeV/u, 200 MeV/u, 250 MeV/u, 300 MeV/u und 350 MeV/u entsprechen.

11. Verfahren nach einem der Ansprüche 9 bis 10,
    **dadurch gekennzeichnet,**
    **daß** die Bestrahlungsdosis mit Ionisationskammern gemessen wird.

12. Verfahren nach einem der Ansprüche 9 bis 11,
    **dadurch gekennzeichnet,**
    **daß** die Bestrahlungsdosis mit Hilfe eines Verifikationsfilms gemessen wird, welcher infolge der jeweiligen Bestrahlung geschwärzt wird, wobei die Schwankungsbreite der sich infolge der Bestrahlung einstellenden Schwärzung des Verifikationsfilms zur Beurteilung der Homogenität der Bestrahlungsdosisverteilung quer zur Strahlrichtung des Schwerionenstrahls (11) ausgewertet wird.

13. Verfahren nach einem der Ansprüche 9 bis 2,
    **dadurch gekennzeichnet,**
    **daß** die Bestrahlung des Phantoms zur Bestimmung der Homogenität der Bestrahlungsdosisverteilung quer zur Strahlrichtung des Schwerionenstrahls (11) mit einer Bestrahlungsdosis von ca. 1 Gy erfolgt .

14. Verfahren nach einem der vorhergehenden Ansprüche,
    **dadurch gekennzeichnet,**
    **daß** der Toleranzgrenzwert für die Schwankungsbreite ±5 % beträgt.

15. Verfahren nach einem der vorhergehenden Ansprüche,
    **dadurch gekennzeichnet,**
    **daß** im Bereich des Isozentrums (10) mindestens ein Strahlparameter des Schwerionenstrahls (11) mit entsprechenden Überwachungsmitteln überwacht wird, wobei die zur Überwachung des mindestens einen Strahlparameter des Schwerionenstrahls (11) vorgesehenen Überwachungsmittel in Übereinstimmung mit einem bestimmten Kalibrierfaktor kalibriert werden, und

**daß** die zeitliche Schwankung des Kalibrierfaktors überwacht wird, wobei auf eine ausreichende zeitliche Konstanz des Kalibrierfaktors der Überwachungsmittel geschlossen wird, falls diese einen bestimmten Toleranzgrenzwert nicht überschreitet.

16. Verfahren nach Anspruch 15,
**dadurch gekennzeichnet,**
**daß** die zur Überwachung des mindestens einen Strahlparameters des Schwerionenstrahls (11) vorgesehenen Überwachungsmittel Ionisationskammern umfassen, welche als Strahlparameter die Teilchenzahl des Schwerionenstrahls (11) überwachen.

17. Verfahren nach einem der Ansprüche 15 bis 6,
**dadurch gekennzeichnet,**
**daß** zur Überprüfung der Konstanz des Kalibrierfaktors der Überwachungsmittel ein Festkörperphantom bestrahlt wird.

18. Verfahren nach Anspruch 15 oder 17,
**dadurch gekennzeichnet,**
**daß** die Konstanz des Kalibrierfaktors der Überwachungsmittel für mehrere unterschiedliche Bestrahlungsenergien überprüft wird, wobei nur dann auf eine ausreichende zeitliche Konstanz des Kalibrierfaktors der Überwachungsmittel geschlossen wird, falls der Toleranzgrenzwert für jede dieser Bestrahlungsenergien eingehalten wird.

19. Verfahren nach einem der Ansprüche 15 bis 18,
**dadurch gekennzeichnet,**
**daß** der Toleranzgrenzwert einer maximalen Schwankung von ±3 % entspricht.

20. Verfahren nach einem der Ansprüche 15 bis 19,
**dadurch gekennzeichnet,**
**daß** der Einfluß der Teilchenfluenz des Schwerionenstrahls (11) auf den Kalibrierfaktor überwacht wird.

21. Verfahren nach Anspruch 20,
**dadurch gekennzeichnet,**
**daß** eine bestimmte Bestrahlungsfläche eines Phantoms bei einer bestimmten Bestrahlungsenergie und konstanter Strahlintensität bestrahlt und die Bestrahlungsdosis in der Mitte der Bestrahlungsfläche erfaßt wird, wobei die Abweichung zwischen der gemessenen Bestrahlungsdosis und einem Referenzwert ermittelt und auf einen Fehler geschlossen wird, falls die Abweichung größer als ±3 % ist.

22. Verfahren nach Anspruch 21,
**dadurch gekennzeichnet,**
**daß** die Bestrahlungsfläche des Phantoms einer Fläche von 5 x 5 cm$^2$ entspricht, die mit den Bestrahlungsenergien 150 MeV/u, 250 MeV/u und 350 MeV/u bestrahlt wird, wobei die Überwachungseinheiten der Überwachungsmittel derart festgelegt werden, daß sich nominal als Referenzwert am Meßort eine Bestrahlungsdosis von 0,2 Gy, 0,5 Gy bzw. 1 Gy einstellt.

23. Verfahren nach einem der Ansprüche 15 bis 22,
**dadurch gekennzeichnet,**
**daß** der Einfluß des Teilchenflusses des Schwerionenstrahls (11) auf den Kalibrierfaktor überwacht wird.

24. Verfahren nach Anspruch 23,
**dadurch gekennzeichnet,**
**daß** mehrere Bestrahlungen mit unterschiedlichen Strahlintensitäten des Schwerionenstrahls (11) durchgeführt werden, wobei die nominale Bestrahlungsdosis konstant gehalten wird, und daß die von den Überwachungsmitteln gemessenen Bestrahlungsdosiswerte mit der nominalen Bestrahlungsdosis verglichen werden, wobei auf das Vorliegen eines Fehlers geschlossen wird. falls für wenigstens eine der Strahlintensitäten eine Abweichung der gemessenen Bestrahlungsdosis von der nominalen Bestrahlungsdosis von mehr als ±3 % auftritt.

25. verfahren nach einem der Ansprüche 13 bis 24,
**dadurch gekennzeichnet,**
**daß** die Abhängigkeit des Kalibrierfaktors von der Strahllage des Schwerionenstrahls (11) überwacht wird.

26. Verfahren nach Anspruch 25,
**dadurch gekennzeichnet,**
**daß** ein bestimmtes Bestrahlungsvolumen durch die Rasterscannereinrichtung (13, 14) derart bestrahlt wird, daß sich in der Mitte des Bestrahlungsvolumens eine bestimmte Bestrahlungsdosis einstellt, wobei die Abweichung der an einer bezüglich der Mitte des Bestrahlungsvolumens seitlichen Position gemessenen Bestrahlungsdosiswerte von der bestimmten Bestrahlungsdosis ermittelt wird, und

**daß** auf das Vorliegen eines Fehlers geschlossen wird, falls die Abweichung größer als ±3 % ist.

27. Verfahren nach Anspruch 26,
**dadurch gekennzeichnet,**
**daß** die bestimmte Bestrahlungsdosis in der Mitte des Bestrahlungsvolumens, welches eine Würfelform mit einer Kantenlänge von ca. 5 cm besitzt, 1 Gy beträgt, und

**daß** die seitlichen Positionen, an denen die Bestrahlungsdosis gemessen wird, um 3 cm und 6 cm bezüglich der Mitte des Bestrahlungsvolumens versetzt sind.

28. verfahren nach einem der vorhergehenden Ansprüche,
   **dadurch gekennzeichnet,**
   **daß** die Konstanz der Bestrahlungsdosis dadurch Überprüft wird, daß ein bestimmtes Bestrahlungsvolumen eines Phantoms durch die Rasterscannereinrichtung (13, 14) bestrahlt wird, wobei sich das Isozentrum (10) in der Mitte der vorderen Oberfläche des Phantoms befindet, und die im Mittelpunkt des Bestrahlungsvolumens auftretende Bestrahlungsdosis als Referenzwert erfaßt und mit den nachfolgend gemessenen Werten der Bestrahlungsdosis verglichen wird.

29. Verfahren nach Anspruch 28,
   **dadurch gekennzeichnet,**
   **daß** auf eine ausreichend genaue Konstanz der Bestrahlungsdosis geschlossen wird, falls die Abweichung zwischen den einzelnen Werten der Bestrahlungsdosis von dem Referenzwert maximal ±3 % beträgt.

30. Verfahren nach Anspruch 27 oder 28,
   **dadurch gekennzeichnet,**
   **daß** das Bestrahlungsvolumen eine würfelform mit einer Kantenlänge von ca. 5 cm besitzt, wobei die Bestrahlungsdosis in einer wasseräquivalenten Tiefe von 11,3 cm gemessen wird.

**Claims**

1. Method of operating a heavy ion beam therapy system under monitoring of the radiation dose distribution,
   wherein the heavy ion beam therapy system comprises

   - at least one ion source (1)
   - an accelerator device (2, 5) for the acceleration of the ions of the ion source (1) in the form of a heavy ion beam (11),
   - a beam guidance system (6, 8), to guide the heavy ion beam (11) from the accelerator device (2, 5) to at least one irradiation site for treatment of a patient, the beam guidance system (6, 8) comprising at least one beam guidance channel (6), and
   - a grid scanner device, arranged in the beam guidance system (6, 8), having vertical deflection means (13) and horizontal deflection means (14) for the vertical and horizontal deflection of the heavy ion beam (11) perpendicular to its beam direction, with the result that the heavy ion beam (11) is deflected by the grid scanner device to an isocentre (10) of the irradiation site and scans a specific area surrounding the isocentre (10) using a specific radiation dose,

   **characterised in that**
   the radiation dose distribution of the grid scanner device (13, 14) at various positions in the region of the isocentre (10) is measured and evaluated; and it is concluded that the radiation dose distribution is adequately homogeneous if the degree of variation in the radiation dose values measured at the individual positions does not exceed a specific tolerance limit value; and monitoring means are calibrated in accordance with a calibration factor K in respect of the particle count, ascertained by those means, per monitoring unit of the monitoring means, the calibration factor K being calculated, as a function of the irradiation energy E of the particles of the heavy ion beam (11) and the scanning step width $\Delta x$ and $\Delta y$ of the grid scanner device (13, 14), by means of :

$$K(E, \Delta x, \Delta y) = (D_{scan}/M_i) \cdot \Delta x \cdot \Delta y/(S(E)/\rho)$$

in which the expression $(S(E)/\rho)$ corresponds to the mass stopping power of the particles of the heavy ion beam (11) at an irradiation energy E,
M corresponding to the number of monitoring units per coordinate point i of the monitoring means, and $D_{scan}$ corresponding to the radiation dose value ascertained by the monitoring means and converted into an energy dose value related to water.

2. Method according to claim 1,
   **characterised in that**
   the radiation dose distribution of the grid scanner device (13, 14) in the beam direction of the heavy ion beam (11) is measured and evaluated.

3. Method according to claim 2,
   **characterised in that**
   irradiation of a phantom is carried out using the grid scanner device (13, 14) by producing irradiation volumes of specific shape, the irradiation being so performed that a homogeneous dose distribution is established in each irradiation volume; and
   the radiation dose is measured at various depth positions of the phantom and compared with a reference value.

4. Method according to claim 3,
   **characterised in that**
   the shape of the irradiation volumes produced by the grid scanner device (13, 14) is parallelepipedal.

5. Method according to claim 4,
**characterised in that**
the irradiation is so performed by the grid scanner device (13, 14) that the edge lengths of the parallelepipedal irradiation volumes are approximately 2.5 cm, 5 cm and 10 cm.

6. Method according to one of claims 3-5,
**characterised in that**
the radiation dose is measured at the various depth positions of the phantom using ionisation chambers.

7. Method according to one of claims 3 to 6,
**characterised in that**
the various depth positions, at which the radiation dose is measured, correspond to a depth of the parallelpipedal irradiation volume of 5 cm, 12.5 cm and 20 cm, respectively.

8. Method according to one of the preceding claims,
**characterised in that**
the radiation dose distribution of the grid scanner device (13, 14) transverse to the beam direction of the heavy ion beam (11) is measured and evaluated.

9. Method according to claim 8,
**characterised in that**
a phantom is irradiated using the grid scanner device (13, 14) by producing irradiation areas, a plurality of irradiations being carried out using various irradiation energies and various irradiation areas and, in each case, the radiation dose being measured perpendicular to the beam direction of the heavy ion beam (11).

10. Method according to claim 9,
**characterised in that,**
the various irradiation energies of the plurality of irradiations correspond to 100 MeV/u, 150 MeV/u, 200 MeV/u, 250 MeV/u, 300 MeV/u and 350 MeV/u.

11. Method according to one of claims 9 to 10,
**characterised in that**
the radiation dose is measured using ionisation chambers.

12. Method according to one of claims 9 to 11,
**characterised in that**
the radiation dose is measured with the aid of a verification film, which is blackened as a result of the respective irradiation, the range of variation in the blackening of the verification film brought about as a result of the irradiation being evaluated for assessing the homogeneity of the radiation dose distribution transverse to the beam direction of the heavy ion beam (11).

13. Method according to one of claims 9 to 12,
**characterised in that**
the irradiation of the phantom for determining the homogeneity of the radiation dose distribution transverse to the beam direction of the heavy ion beam (11) is performed using a radiation dose of approximately 1 Gy.

14. Method according to one of the preceding claims,
**characterised in that**
the tolerance limit value for the range of variation is ±5 %.

15. Method according to one of the preceding claims,
**characterised in that**,
in the region of the isocentre (10), at least one beam parameter of the heavy ion beam (11) is monitored using appropriate monitoring means, the monitoring means provided for monitoring the at least one beam parameter of the heavy ion beam (11) being calibrated in accordance with a specific calibration factor; and
the variation in the calibration factor over time is monitored, it being concluded that the consistency of the calibration factor of the monitoring means over time is adequate if it does not exceed a specific tolerance limit value.

16. Method according to claim 15,
**characterised in that**
the monitoring means provided for monitoring the at least one beam parameter of the heavy ion beam (11) comprise ionisation chambers, which monitor, as the beam parameter, the particle count of the heavy ion beam (11).

17. Method according to one of claims 15 to 16,
**characterised in that**
for checking the consistency of the calibration factor of the monitoring means, a solid-body phantom is irradiated.

18. Method according to claim 15 or 17,
**characterised in that,**
the consistency of the calibration factor of the monitoring means is checked for a plurality of different irradiation energies, it being concluded that the consistency of the calibration factor of the monitoring means over time is adequate only if the tolerance limit value is met for each of those irradiation energies.

19. Method according to one of claims 15 to 18,
**characterised in that**
the tolerance limit value corresponds to a maximum variation of ±3 %.

20. Method according to one of claims 15 to 19,

**characterised in that**
the influence of the particle fluence of the heavy ion beam (11) on the calibration factor is monitored.

21. Method according to claim 20,
**characterised in that**
a specific irradiation area of a phantom is irradiated at a specific irradiation energy and constant beam intensity and the radiation dose in the middle of the irradiation area is ascertained, the deviation between the measured radiation dose and a reference value being determined and it being concluded that there is an error if the discrepancy is greater than ±3 %.

22. Method according to claim 21,
**characterised in that**
the irradiation area of the phantom corresponds to a surface area of 5 x 5 cm$^2$ which is irradiated at irradiation energies of 150 MeV/u, 250 MeV/u and 350 MeV/u, the monitoring units of the monitoring means being so set that nominally, as a reference value, a radiation dose of 0.2 Gy, 0.5 Gy and 1 Gy, respectively, is brought about at the measurement site.

23. Method according to one of claims 15 to 22,
**characterised in that**
the influence of the particle flow of the heavy ion beam (11) on the calibration factor is monitored.

24. Method according to claim 23,
**characterised in that**
a plurality of irradiations are carried out using different beam intensities of the heavy ion beam (11), the nominal radiation dose being kept constant; and the radiation dose values measured by the monitoring means are compared with the nominal radiation dose, it being concluded that an error is present if, for at least one of the beam intensities, there is a deviation of the measured radiation dose from the nominal radiation dose of more than ±3 %.

25. Method according to one of claims 15 to 24,
**characterised in that**
the dependence of the calibration factor on the beam position of the heavy ion beam (11) is monitored.

26. Method according to claim 25,
**characterised in that**
a specific irradiation volume is so irradiated by the grid scanner device (13, 14) that a specific radiation dose is brought about in the centre of the irradiation volume, the deviation of the radiation dose values measured at a lateral position with respect to the centre of the irradiation volume from the specific radiation dose being determined; and

it is concluded that an error is present if the deviation is greater than ±3 %.

27. Method according to claim 26,
**characterised in that**
the specific radiation dose in the centre of the irradiation volume, which is in the shape of a cube having an edge length of approximately 5 cm, is 1 Gy; and
the lateral positions at which the radiation dose is measured are 3 cm and 6 cm from the centre of the irradiation volume.

28. Method according to one of the preceding claims,
**characterised in that**
the consistency of the radiation dose is checked by irradiating a specific irradiation volume of a phantom by means of the grid scanner device (13, 14), the isocentre (10) being located in the centre of the front face of the phantom, and the radiation dose occurring in the centre point of the irradiation volume being ascertained as a reference value and compared with the subsequently measured values of the radiation dose.

29. Method according to claim 28,
**characterised in that**
it is concluded that the consistency of the radiation dose is adequately accurate if the deviation between the individual values of the radiation dose and the reference value is maximally ±3 %.

30. Method according to claim 27 or 28,
**characterised in that**
the irradiation volume has a cube shape having an edge length of approximately 5 cm, the radiation dose being measured at a water-equivalent depth of 11.3 cm.

**Revendications**

1. Procédé permettant de faire fonctionner un système de thérapie par faisceaux d'ions lourds en surveillant la répartition de la dose de rayonnement, le système de thérapie par faisceaux d'ions lourds comprenant :

   • au moins une source d'ions (1),
   • un dispositif d'accélération (2, 5) pour accélérer les ions de la source d'ions (1) sous la forme d'un faisceau d'ions lourds (11),
   • un système de guidage de faisceau (6, 8) pour amener le faisceau d'ions lourds (11) du dispositif d'accélération (2, 5) à au moins un poste d'irradiation pour le traitement d'un patient, le système de guidage de faisceau (6, 8) comprenant au moins un conduit de guidage de faisceau (6), et

- un système de scanner à balayage disposé dans le système de guidage de faisceau (6, 8), avec des moyens de déviation verticale (13) et des moyens de déviation horizontale (14) pour dévier verticalement voire horizontalement le faisceau d'ions lourds (11) perpendiculairement à sa direction de faisceau, de manière telle que le faisceau d'ions lourds soit dévié par le système de scanner à balayage sur un isocentre (10) du poste d'irradiation et balaie avec une certaine dose de rayonnement une surface déterminée autour de l'isocentre (10),

**caractérisé par le fait qu'**on mesure et on calcule la répartition de la dose de rayonnement du système de scanner à balayage (13, 14) en différents emplacements dans la région de l'isocentre (10) et

on considère que la répartition de la dose de rayonnement est suffisamment homogène lorsque l'amplitude de fluctuation des valeurs de dose de rayonnement mesurées aux différents emplacements ne dépasse pas une valeur limite de tolérance déterminée et par le fait qu'on étalonne des moyens de surveillance en accord avec un facteur d'étalonnage K en ce qui concerne le nombre de particules détectées par lesdits moyens par unité de surveillance des moyens de surveillance, le facteur d'étalonnage K, en tant que fonction de l'énergie de rayonnement E des particules du faisceau d'ions lourds (11) et de la longueur de pas de balayage $\Delta x$, $\Delta y$ du système de scanner à balayage (13, 14), étant calculé par la formule :

$$K(E, \Delta x, \Delta y) = (D_{scan}/M_i) * \Delta x * \Delta y/(S(E)/p),$$

l'expression $S(E)/p$ correspondant au pouvoir d'arrêt massique des particules du faisceau d'ions lourds (11) pour l'énergie de rayonnement E,
M correspondant au nombre d'unités de surveillance par point de coordonnées i des moyens de surveillance et
$D_{scan}$ correspondant à la valeur de dose de rayonnement mesurée par les moyens de surveillance convertie en une valeur de dose d'énergie dans l'eau.

2. Procédé selon la revendication 1, **caractérisé par le fait qu'**on mesure et on calcule la répartition de la dose de rayonnement du système de scanner à balayage (13, 14) dans la direction de faisceau du faisceau d'ions lourds (11).

3. Procédé selon la revendication 2, **caractérisé par le fait qu'**on procède à une irradiation d'un fantôme à l'aide le système de scanner à balayage (13, 14) en produisant des volumes d'irradiation de forme déterminée, l'irradiation étant réalisée de manière à obtenir une répartition homogène de la dose dans chaque volume d'irradiation et **par le fait qu'**on mesure la dose de rayonnement en différentes positions en profondeur du fantôme et on la compare à une valeur de référence.

4. Procédé selon la revendication 3, **caractérisé par le fait que** la forme des volumes d'irradiation produits avec le système de scanner à balayage (13, 14) est celle d'un parallélépipède.

5. Procédé selon la revendication 4, **caractérisé par le fait qu'**on réalise l'irradiation avec le système de scanner à balayage (13, 14) de manière telle que la longueur des arêtes des volumes d'irradiation parallélépipédiques soit d'environ 2,5 cm, 5 cm et 10 cm.

6. Procédé selon une des revendications 3 à 5, **caractérisé par le fait qu'**on mesure la dose de rayonnement aux différentes positions en profondeur du fantôme à l'aide de chambres d'ionisation.

7. Procédé selon une des revendications 3 à 6, **caractérisé par le fait que** les différentes positions en profondeur auxquelles on mesure la dose de rayonnement correspondent à une profondeur de 5 cm, 12,5 cm voire 20 cm du volume d'irradiation parallélépipédique.

8. Procédé selon une des revendications précédentes, **caractérisé par le fait qu'**on mesure et on calcule la répartition de la dose de rayonnement du système de scanner à balayage (13, 14) transversalement à la direction du faisceau d'ions lourds (11).

9. Procédé selon la revendication 8, **caractérisé par le fait qu'**on procède à une irradiation d'un fantôme à l'aide du système de scanner à balayage (13, 14) en produisant des surfaces d'irradiation, plusieurs irradiations avec des énergies de rayonnement différentes et des surfaces d'irradiation différentes étant réalisées et la dose de rayonnement étant mesurée chaque fois perpendicualirement à la direction de faisceau du faisceau d'ions lourds (11).

10. Procédé selon la revendication 9, **caractérisé par le fait que** les énergies de rayonnement des différentes irradiations correspondent à 100 MeV/u, 150 MeV/u, 200 MeV/u, 250 MeV/u, 300 MeV/u et 350 MeV/u.

11. Procédé selon une des revendications 9 à 10, **caractérisé en ce qu'**on mesure la dose de rayonnement à l'aide de chambres d'ionisation.

**12.** Procédé selon une des revendications 9 à 11, **caractérisé en ce qu'**on mesure la dose de rayonnement à l'aide d'un film de contrôle, lequel film, du fait de l'irradiation, est noirci, l'amplitude de fluctuation du noircissement du film de contrôle consécutif à l'irradiation étant utilisée pour apprécier l'homogénéité de la répartition de la dose de rayonnement transversalement à la direction de faisceau du faisceau d'ions lourds (11).

**13.** Procédé selon une des revendications 9 à 12, **caractérisé en ce que** l'irradiation du fantôme pour déterminer l'homogénéité de la répartition de la dose de rayonnement transversalement à la direction de faisceau du faisceau d'ions lourds (11) a lieu avec une dose de rayonnement d'environ 1 Gy.

**14.** Procédé selon une des revendications précédentes, **caractérisé par le fait que** la valeur limite de tolérance pour l'amplitude de fluctuation est $\pm 5\%$.

**15.** Procédé selon une des revendications précédentes, **caractérisé par le fait que** dans la région de l'isocentre (10), on surveille au moins un paramètre de faisceau du faisceau d'ions lourds (11), le moyen de surveillance prévu pour la surveillance du paramètre de faisceau, au nombre d'au moins un, du faisceau d'ions lourds (11), étant étalonné en accord avec facteur d'étalonnage déterminé et **par le fait qu'**on surveille la fluctuation dans le temps du facteur d'étalonnage, la constance dans le temps du facteur d'étalonnage du moyen de surveillance étant considérée suffisante, lorsque celle-ci ne dépasse pas une valeur limite de tolérance déterminée.

**16.** Procédé selon la revendication 15, **caractérisé par le fait que** les moyens de surveillance prévus pour la surveillance du paramètre de faisceau, au nombre d'au moins un, du faisceau d'ions lourds (11), comprend des chambres d'ionisation qui surveillent, comme paramètre de faisceau, le nombre de particules du faisceau d'ions lourds (11).

**17.** Procédé selon une des revendications 15 à 16, **caractérisé par le fait que** pour contrôler la constance du facteur d'étalonnage des moyens de surveillance on irradie un fantôme solide.

**18.** Procédé selon la revendication 15 ou 17, **caractérisé par le fait qu'**on contrôle la constance du facteur d'étalonnage des moyens de surveillance pour différentes énergies de rayonnement, la constance dans le temps du facteur d'étalonnage des moyens de surveillance n'étant considérée suffisante que lorsque la valeur limite de tolérance pour chacune desdites énergies de rayonnement est respectée.

**19.** Procédé selon une des revendications 15 à 18, **caractérisé par le fait que** la valeur limite de tolérance correspond à une fluctuation maximale de $\pm 3\%$.

**20.** Procédé selon une des revendications 15 à 19, **caractérisé par le fait qu'**on contrôle l'influence de la fluence de particules du faisceau d'ions lourds (11) sur la facteur d'étalonnage.

**21.** Procédé selon la revendication 20, **caractérisé par le fait qu'**on irradie une surface d'irradiation déterminée d'un fantôme avec une énergie de rayonnement déterminée et une intensité de rayonnement constante, on mesure la dose de rayonnement au centre de la surface d'irradiation, on détermine l'écart entre la dose de rayonnement mesurée et une valeur de référence et on considère qu'il existe une erreur lorsque l'écart est supérieur à $\pm 3\%$.

**22.** Procédé selon la revendication 21, **caractérisé par le fait que** la surface d'irradiation du fantôme correspond à une surface de 5*5 cm$^2$ qui est irradiée avec les énergies de rayonnement 150 MeV/u, 250 MeV/u et 350 MeV/u, les unités de surveillance des moyens de surveillance étant fixée de manière à obtenir en nominal comme valeur de référence au point de mesure une dose de rayonnement de 0,2 Gy, 0,5 Gy et 1 Gy.

**23.** Procédé selon une des revendications 15 à 22, **caractérisé par le fait qu'**on surveille l'influence du flux de particules du faisceau d'ions lourds (11) sur le facteur d'étalonnage.

**24.** Procédé selon la revendication 23, **caractérisé par le fait qu'**on procède à plusieurs irradiations avec différentes intensités de faisceau du faisceau d'ions lourds (11), la dose de rayonnement nominale étant maintenue constante, et **par le fait qu'**on compare les valeurs de dose de rayonnement mesurées par les moyens de surveillance à la dose de rayonnement nominale et on conclut à la présence d'une erreur lorsque pour au moins une des intensités de rayonnement l'écart entre les doses de rayonnement mesurées par rapport à la dose de rayonnement nominale est supérieur à $\pm 3\%$.

**25.** Procédé selon une des revendications 15 à 24, **caractérisé par le fait qu'**on surveille la relation de dépendance du facteur d'étalonnage de la position du faisceau d'ions lourds (11).

**26.** Procédé selon la revendication 25, **caractérisé par le fait qu'**on irradie un volume d'irradiation déterminé à l'aide du système de scanner à balayage (13, 14) de manière à obtenir au centre du volume d'irradiation une dose de rayonnement déterminée, on calcule l'écart entre les valeurs de dose de rayonnement mesurées dans une position latérale par rapport au centre du volume d'irradiation et la dose de rayonnement déterminée et qu'on considère qu'une erreur est présente lorsque l'écart est supérieur à ±3%.

27. Procédé selon la revendication 26, **caractérisé par le fait que** la dose de rayonnement déterminée au centre du volume d'irradiation, dont la forme est celle d'un cube avec une longueur d'arête d'environ 5 cm, est de 1 Gy et que les positions latérales au niveau desquelles on mesure la dose de rayonnement sont à 3 cm et 6 cm par rapport au centre du volume d'irradiation.

28. Procédé selon une des revendications précédentes **caractérisé par le fait qu'**on contrôle la constance de la dose de rayonnement en irradiant un volume d'irradiation déterminé d'un fantôme à l'aide du système de scanner à balayage (13, 14), l'isocente (10) étant situé au centre de la surface antérieure du fantôme, on mesure en tant que valeur de référence la dose de rayonnement apparaissant au centre du volume d'irradiation et on la compare aux valeurs mesurées par la suite de la dose de rayonnement.

29. Procédé selon la revendication 28, **caractérisé par le fait qu'**on considère que la dose de rayonnement est suffisamment constante lorsque l'écart entre les différentes valeurs de la dose de rayonnement et la valeur de référence est au maximum de ±3%.

30. Procédé selon la revendication 27 ou 28, **caractérisé par le fait que** le volume d'irradiation a une forme de cube avec une longueur d'arête d'environ 5 cm, la dose de rayonnement étant mesurée à une profondeur équivalente d'eau de 11,3 cm.

## Fig. 1

Fig. 2

EP 1 152 794 B1

Fig. 3A

Positionsregelung: Soll/Istwerte ohne Regelung

Fig. 3B

Positionsregelung: Soll/Istwerte mit Regelung

## Positionsregelung: Soll/Istwerte ohne Regelung

**Fig.4A**

## Positionsregelung: Soll/Istwerte mit Regelung

**Fig.4B**